(19) Europäisches Patentamt
European Patent Office
Office européen des brevets

(11) EP 3 395 910 A1

(12) **EUROPEAN PATENT APPLICATION**
published in accordance with Art. 153(4) EPC

(43) Date of publication:
**31.10.2018 Bulletin 2018/44**

(21) Application number: **16878810.7**

(22) Date of filing: **21.12.2016**

(51) Int Cl.:
***C08L 101/02*** *(2006.01)* ***C12M 3/00*** *(2006.01)*
***C12N 5/07*** *(2010.01)*

(86) International application number:
**PCT/JP2016/088209**

(87) International publication number:
**WO 2017/110923 (29.06.2017 Gazette 2017/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.12.2015 JP 2015251518**

(71) Applicant: **AGC Inc.**
**Tokyo 100-8405 (JP)**

(72) Inventors:
- **SATO, Hiroki**
  **Tokyo 100-8405 (JP)**
- **MIWA, Tatsuaki**
  **Tokyo 100-8405 (JP)**
- **IDIRIS, Alimjan**
  **Tokyo 100-8405 (JP)**
- **KOGUCHI, Ryohei**
  **Tokyo 100-8405 (JP)**
- **YAMAMOTO, Kyoko**
  **Tokyo 100-8405 (JP)**
- **EGUCHI, Hajime**
  **Tokyo 100-8405 (JP)**

(74) Representative: **Müller-Boré & Partner Patentanwälte PartG mbB**
**Friedenheimer Brücke 21**
**80639 München (DE)**

# (54) RESIN COMPOSITION, SUBSTRATE, AND CELL CULTURE METHOD

(57) To provide a resin composition which is used to coat the substrate surface, thereby to selectively trap a variety of target substances via a ligand which specifically binds to the target substances on the substrate surface.

The resin composition of the present invention comprises a polymer having units (a) having at least one functional group selected from the group consisting of a maleimide group, a succinimide group, a thiol group and a hydrazino group, and units (b) having at least one group selected from the group consisting of a group represented by the following formula (1), a group represented by the following formula (2) and a group represented by the following formula (3), or comprises a polymer (A) having units (a) having at least one functional group selected from the group consisting of a maleimide group, a succinimide group, a thiol group and a hydrazino group, and a polymer (B) having units (b) having at least one group selected from the group consisting of a group represented by the following formula (1), a group represented by the following formula (2) and a group represented by the following formula (3):

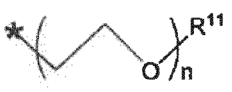 (1)

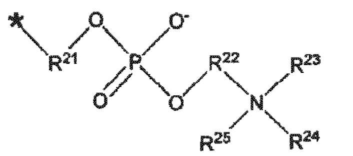 (2)

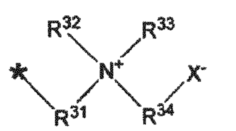 (3)

EP 3 395 910 A1

Fig. 3

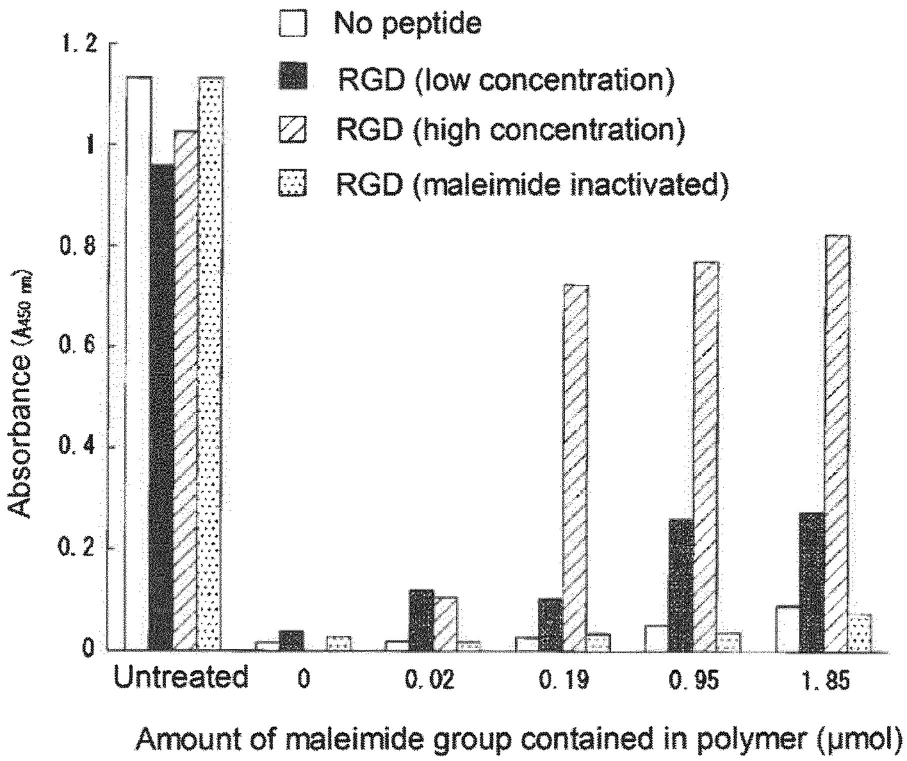
No peptide
RGD (low concentration)
RGD (high concentration)
RGD (maleimide inactivated)
Absorbance (A450 nm)
1.2
1
0.8
0.6
0.4
0.2
0
Untreated
0
0.02
0.19
0.95
1.85
Amount of maleimide group contained in polymer (μmol)

**Description**

TECHNICAL FIELD

The present invention relates to a resin composition suitable particularly as a coating material for e.g. a substrate to selectively trap specific cells, a substrate, and a cell culture process.

BACKGROUND ART

Synthetic polymer materials such as hydrophobic polymers (such as polyvinyl chloride, polystyrene, a silicone resin, a polymethacrylic acid ester and a fluororesin) and hydrophilic polymers (such as polyvinyl alcohol, poly(2-hydroxyethyl methacrylate) and polyacrylamide) are widely used as medical materials. For example, medical devices such as a cell culture vessel, a catheter, an artificial organ and a carrier for affinity purification have been known.

However, such synthetic polymer materials are insufficient in biocompatibility in many cases. That is, proteins such as fibrinogen, immunoglobulin G (IgG), insulin, histone and carbonic anhydrase are likely to be adsorbed on the device surface. Once such proteins are adsorbed on the device surface, further cells (such as blood cells and blood platelets) are likely to adhere to that portion, thus causing harmful effects on a living body such as thrombus formation and inflammatory reaction, and deterioration of the device.

Accordingly, for a medical device using such a synthetic polymer material, a coating layer made of a synthetic polymer material such as a polymer of 2-methacryloyloxyethyl phosphorylcholine having a structure similar to a biological membrane, or a polymer containing polyoxyethylene glycol, is formed on the surface to improve biocompatibility.

Further, in recent years, as a cell culture vessel to be used for multipotent stem cells, a technique to coat the culture vessel with proteins extracted from mouse sarcoma cells has been developed (Non-Patent Document 1). However, in the field of regenerative medicine, impurities such as heterozoic animal-derived or nonself-derived cells, serum and proteins may have unexpected harmful effects to a human body. In order to eliminate the risk caused by such non-specified factors, a vessel containing no heterozoic animal-derived or nonself-derived components, having a definite composition, and capable of incubating multipotent stem cells has been desired.

Patent Document 1 discloses a cell culture article obtained by polymerizing e.g. a (meth)acrylate monomer having a carboxy group and laminating the resulting polymer on the substrate surface and bonding cell-adhesive peptides to the carboxy groups by a covalent bond. In the cell culture article, eukaryotic cells including stem cells and undifferentiated stem cells can be fixed via the peptides and incubated.

PRIOR ART DOCUMENTS

PATENT DOCUMENTS

Patent Document 1: JP-A-2011-510655

NON-PATENT DOCUMENTS

Non-Patent Document 1: Xu, Chunhui., et al., Feeder-free growth of undifferentiated human embryonic stem cells, Nat. Biotech., 19(10), 971-979, 2001.

DISCLOSURE OF INVENTION

TECHNICAL PROBLEM

The cell culture article as disclosed in Patent Document 1 may have harmful effects on cell culture since cell-derived proteins, etc. may be non-specifically adsorbed on the substrate surface. Further, only functional groups capable of being covalently bonded to a carboxy group (e.g. amino groups) can be fixed.

Under these circumstances, it is an object of the present invention to provide a resin composition which can selectively trap a wide variety of target substances on the surface of a substrate, by coating the substrate surface, via a ligand which specifically binds to the target substances, while non-specific adsorption of proteins, etc. is suppressed.

SOLUTION TO PROBLEM

The present invention provides the following.

[1] A resin composition comprising a polymer having units (a) having at least one functional group selected from the group consisting of a maleimide group, a succinimide group, a thiol group and a hydrazino group, and units (b) having at least one group selected from the group consisting of a group represented by the following formula (1), a group represented by the following formula (2) and a group represented by the following formula (3), or comprising a polymer (A) having the units (a) and a polymer (B) having the units (b):

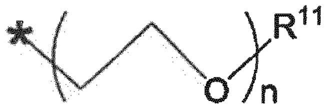
(1)

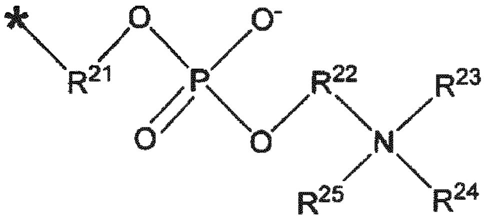
(2)

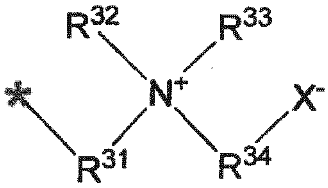
(3)

wherein * is a direct binding site to the polymer main chain or an indirect binding site via a linking group; n is an integer of from 1 to 10, $R^{11}$ is a hydrogen atom, a methyl group or an ethyl group; $R^{21}$ and $R^{22}$ are each independently a $C_{1-5}$ alkylene group, and $R^{23}$ to $R^{25}$ are each independently a $C_{1-5}$ alkyl group; $R^{31}$ is a $C_{1-20}$ alkylene group, $R^{34}$ is a $C_{1-5}$ alkylene group, $R^{32}$ and $R^{33}$ are each independently a $C_{1-5}$ alkyl group, and $X^-$ is a group represented by the following formula (4) or a group represented by the following formula (5):

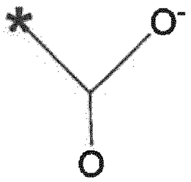
(4)
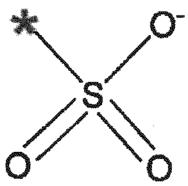
(5)

wherein * is a binding site to $R^{34}$.

[2] The resin composition according to [1], which further contains a polymer (C) having units (c) having a group represented by the following formula (6), or wherein the polymer having the units (a) and the units (b) has units (c) having a group represented by the following formula (6), or at least one of the polymer (A) and the polymer (B) has units (c) having a group represented by the following formula (6):

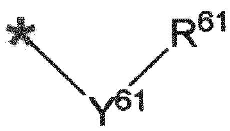
(6)

wherein * is a direct binding site to the polymer main chain, $Y^{61}$ is a single bond or a bivalent organic group, and $R^{61}$ is a $C_{1-20}$ alkyl group.

[3] The resin composition according to [1] or [2], wherein in the polymer having the units (a) and the units (b) or in the polymer (A), the proportion of the units (a) is from 0.001 to 5 mol% to the total number of units constituting the composition.

[4] The resin composition according to any one of [1] to [3], wherein in the polymer having the units (a) and the units (b) or in the polymer (B), the proportion of the units (b) is from 5 to 60 mass% to the total number of units constituting the composition.

[5] The resin composition according to any one of [2] to [4], wherein the units (c) having a group represented by the formula (6) are contained in a proportion of from 30 to 90 mass% to the total number of units constituting the composition.

[6] The resin composition according to any one of [2] to [5], which further contains units (d) having a crosslinkable group selected from a functional group which forms a silanol group by hydrolysis, an epoxy group, a (meth)acrylic group and a glycidyl group.

[7] The resin composition according to [6], which contains from 0.002 to 3 mass% of the units (a), from 10 to 45 mass% of the units (b), from 50 to 80 mass% of the units (c) and from 0 to 2.5 mass% of the units (d).

[8] A substrate which has at least a part of its surface coated with the resin composition as defined in any one of [1] to [7].

[9] The substrate according to [8], which is for cell culture.

[10] A cell culture process, which comprises a step of bonding a ligand having a moiety which specifically binds to the surface of target cells, to the at least one functional group selected from the group consisting of a maleimide group, a succinimide group, a thiol group and a hydrazino group on the surface of the substrate as defined in [8],

a step of bringing the target cells into contact with the substrate having the ligand bonded thereto, to bond the target cells to the ligand, and
a step of incubating the target cells bonded to the ligand.

## ADVANTAGEOUS EFFECTS OF INVENTION

A substrate the surface of which is coated with the resin composition of the present invention, can selectively trap on its surface a target substance via a ligand which specifically binds to the substance and which has a functional group capable of being covalently bonded to a functional group in the units (a), while non-specific adsorption of substances other than the target substance is suppressed.

## BRIEF DESCRIPTION OF DRAWINGS

Fig. 1 is a graph illustrating the relation between the reaction time and the proportion of remaining RGD peptide (containing cysteine) in the 24-well microplate obtained in each of Ex. 1 to 3 in Test Example 3.
Fig. 2 is an image of a state of a plate after cell adhesion assay using TIG-3 cells in Test Example 4.
Fig. 3 is a graph illustrating the results of quantitative determination of the number of bonded cells under each test conditions in Test Example 4.
Fig. 4 is images illustrating comparison of forms of TIG-3 cells between in a well to which high concentration RGD peptide (containing cysteine) is fixed and in an untreated well in Test Example 4.

## DESCRIPTION OF EMBODIMENTS

The following definitions of terms and expression are applicable throughout Description and Claims.

A "monomer represented by the formula (7)" will sometimes be referred to as a "monomer (7)". Monomers represented by other formulae will be referred to in the same manner.

A "group represented by the formula (1)" will sometimes be referred to as a "group (1)". Groups represented by other formulae will be referred to in the same manner.

A "halogen atom" means a fluorine atom, a chlorine atom, a bromine atom or an iodine atom, and is preferably a chlorine atom or a fluorine atom.

A "unit" means a moiety (polymer unit) derived from a monomer present in a polymer and constituting the polymer. A unit derived from a monomer having a carbon-carbon unsaturated double bond formed by addition polymerization of the monomer, is a bivalent unit formed by cleavage of the unsaturated double bond. Further, one obtained by chemically converting the structure of a certain unit after formation of a polymer will also be referred to as a unit. In the following, in some cases, a unit derived from an individual monomer may be referred to by a name having "unit" attached to the monomer's name.

A "(meth)acrylate" is a generic term for an acrylate and a methacrylate.

A "biocompatible group" means a group having a property of inhibiting adsorption of protein on a polymer and adhesion and fixing of cells on a polymer.

The term "biocompatibility" means a property not to let a biological sample such as protein be adsorbed, or not to let cells adhere.

A "protein" means an oligopeptide, a polypeptide and a fragment thereof. It may be a naturally derived protein or may be an artificially synthesized protein and is not limited, and is preferably a naturally derived protein which has less harmful influence such as cytotoxicity in many cases.

A "cell" is the most fundamental unit constituting a living body and means one which has, in the interior of the cell membrane, the cytoplasm and various organelles. Nuclei containing DNA may be contained or may not be contained inside the cell.

Animal-derived cells include germ cells (sperm, ova, etc.), somatic cells constituting a living body, stem cells, progenitor cells, cancer cells separated from a living body, cells (cell line) which are separated from a living body and have won immortalized ability and thus are stably maintained outside the body, cells separated from a living body and artificially genetically engineered, cells separated from a living body and having nuclei artificially replaced, etc.

Somatic cells constituting a living body include fibroblasts, bone marrow cells, B lymphocytes, T lymphocytes, neutrophils, erythrocytes, platelets, macrophages, monocytes, bone cells, bone marrow cells, pericytes, dendritic cells, keratinocytes, fat cells, mesenchymal cells, epithelial cells, epidermal cells, endothelial cells, vascular endothelial cells, hepatocytes, cartilage cells, cumulus cells, neural cells, glial cells, neurons, oligodendrocytes, microglia, astrocytes, cardiac cells, esophagus cells, muscle cells (for example, smooth muscle cells, skeletal muscle cells), pancreatic beta cells, melanin cells, hematopoietic progenitor cells, mononuclear cells, etc.

The somatic cells include cells taken from optional tissues, such as skin, kidneys, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood, heart, eye, brain, nervous tissue, etc.

The stem cells are cells having both an ability to replicate themselves and an ability to be differentiated into cells of other multiple systems, and include embryonic stem cells (ES cells), embryonic carcinoma cells, embryonic germ stem cells, induced pluripotent stem cells (iPS cells), neural stem cells, hematopoietic stem cells, mesenchymal stem cells, liver stem cells, pancreatic stem cells, muscle stem cells, germ stem cells, intestinal stem cells, cancer stem cells, hair follicle stem cells, etc.

The progenitor cells are cells at an intermediate stage during differentiation into specific somatic or germ cells from the stem cells.

The cancer cells are cells that have acquired an unlimited proliferative capacity as derived from somatic cells.

A cell line is cells which have acquired an unlimited proliferative capacity by an artificial manipulation in vitro, and includes HCT116, Huh7, HEK293 (human embryonic kidney cells), HeLa (human cervical carcinoma cell line), HepG2 (human liver cancer cell line), UT7/TPO (human leukemia cell line), CHO (Chinese hamster ovary cell line), MDCK, MDBK, BHK, C-33A, HT-29, AE-1, 3D9, Ns0/1, Jurkat, NIH3T3, PC12, S2, Sf9, Sf21, High Five, Vero, etc.

A "ligand" means a substance capable of specifically binding to a target substance. The ligand may, for example, be a protein, a sugar chain, a lipid complex or a low molecular weight compound.

<Resin composition>

The present invention provides a resin composition comprising a polymer having units (a) having at least one functional group selected from the group consisting of a maleimide group, a succinimide group, a thiol group and a hydrazino group, and units (b) having at least one group selected from the group consisting of a group represented by the following formula (1), a group represented by the following formula (2) and a group represented by the following formula (3), or comprising a polymer (A) having the units (a) and a polymer (B) having the units (b).

The resin composition of the present invention (hereinafter sometimes referred to as a specific resin composition) may be a resin composition comprising a copolymer having units (a) and units (b), or may be a resin composition comprising the polymer (A) and the polymer (B). Further, the specific resin composition may contain units (c) and units (d) other than the units (a) and the units (b).

The specific resin composition is preferably a resin composition comprising the polymer (A) and the polymer (B) in that the proportion of the respective units will readily be adjusted. The polymer (A) may have only the units (a) or may have other units (c) or (d). The polymer (A) preferably has the units (a) and the units (c) in that the water-insolubility of the specific resin composition tends to improve. Further, the polymer (B) may have only the units (b) or may have other units (c) or units (d). The polymer (B) preferably has the units (b) and the units (c) in that the water-insolubility of the specific resin composition tends to improve. That is, the specific resin composition is preferably a mixture of a polymer (A) having the units (a) and the units (c) and a polymer (B) having the units (b) and the units (c).

The respective symbols in the above formulae (1) to (3) are as defined above. Particularly, n is preferably from 1 to 5. $R^{11}$ is preferably a methyl group or an ethyl group. $R^{21}$ and $R^{22}$ are preferably an ethylene group. $R^{23}$ to $R^{25}$ are preferably a methyl group. $R^{31}$ and $R^{34}$ are preferably an ethylene group. $R^{32}$ and $R^{33}$ are preferably a methyl group. $X^-$ is preferably $-SO_3^-$.

In the present invention, a fixing group means at least one functional group selected from the group consisting of a maleimide group, a succinimide group, a thiol group and a hydrazino group.

The fixing group in the units (a) is a group having a role to bind to other molecules which many of biological molecules have, specifically, a group capable of being covalently bonded to an amino group, a thiol group, a carboxy group and an aldehyde group. The specific resin composition can irreversibly bind to a wide variety of substances by the fixing group in the structure (a).

Further, since the specific resin composition has the units (b) having a biocompatible group, non-specific adsorption of biological molecules such as substances other than the substance which specifically binds to the fixing group, particularly protein, is suppressed.

In order to selectively incubate only desired cells (target cells) from a sample containing a plurality of cells and various biomolecules such as a biological sample collected from the body, it is necessary to selectively trap only the target cells in a cell culture vessel and to suppress non-specific adsorption of other biomolecules to the cell culture vessel. The specific resin composition can irreversibly bind to a ligand which specifically binds to the target substance by fixing groups and in addition, can suppress non-specific adsorption of protein by biocompatible groups. Accordingly, it is possible to separate and selectively trap the target substance in the biological sample from other biomolecules such as proteins, by bringing the biological sample into contact with a substrate having its surface coated with the specific resin composition and having a ligand to the desired target substance to be selectively trapped, covalently bonded to the fixing groups on its surface.

The substrate for cell culture having its surface coated with the specific resin composition, is suitable as a cell culture vessel to incubate only specific target cells as separated from other biomolecules such as protein derived from the biological sample.

The specific resin composition preferably has units derived from an ethylene unsaturated polymerizable monomer as a basic structure. The ethylene unsaturated polymerizable monomer may, for example, be a monomer unit capable of constituting a resin such as a (meth)acrylic resin, an olefin resin (such as polyethylene or polypropylene), a styrene resin (such as polystyrene, an acrylonitrile/styrene copolymer or a methyl methacrylate/styrene copolymer), a chlorinated resin (such as polyvinyl chloride or polyvinylidene chloride) or a fluorinated resin.

In the specific resin composition, the polymer having the units (a) may be a polymer having one member selected from units derived from the ethylene unsaturated polymerizable monomer as a basic structure, or may be a polymer having a plural types of monomer units in combination. The same applies to polymers having the units (b), the units (c) or the units (d).

«Unit (a)»

In the specific resin composition, the unit (a) has at least one member selected from the group consisting of a maleimide group, a succinimide group, a thiol group and a hydrazino group as the fixing group. The fixing group in the unit (a) is a group having a role to bind to other molecules which many of biological molecules have, specifically, a group capable of being covalently bonded to an amino group, a thiol group, a carboxy group and an aldehyde group. The resin composition can irreversibly bind to a ligand to a wide variety of target substances by the fixing group in the structure (a).

In the specific resin composition, the units (a) may have a single type of fixing groups or may have two or more types of fixing groups in combination. For example, in a case where there are two or more types of target substances, the specific resin composition may have two types of the units (a) (for example, units (a) having a maleimide group and units (a) having a succinimide group) so that two types of ligands can be bonded.

In a case where the fixing group is a maleimide group, a ligand having a cysteine residue can be fixed. In a case where the fixing group is a succinimide group, a ligand having an amino group can be fixed. In a case where the fixing group is a thiol group, a ligand having a carboxy group can be fixed. In a case where the fixing group is a hydrazino group, a ligand having an aldehyde group can be fixed. Particularly, the fixing group is preferably a succinimide group or a thiol group, in view of high general purpose property with respect to the ligand to be fixed.

The monomer from which the units (a) having the fixing group are derived may, for example, be a monomer represented by the following formula (7):

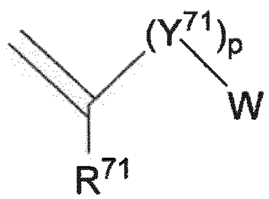

(7)

In the monomer (7), $R^{71}$ is a hydrogen atom, a halogen atom or a methyl group. $Y^{71}$ is a single bond, -O-, -S-, -NH-, -CO-, -COO-, -CONH-, a $C_{1-10}$ alkylene group or a $C_{1-10}$ alkylene glycol group, and p is an integer of from 1 to 10. A plurality of $Y^{71}$'s present in one molecule of the monomer (7) (when p is 2 or more) may be the same or different. W is a maleimide group, a succinimide group, a thiol group or a hydrazino group.

In the monomer (7), a $R^{71}$ is preferably a hydrogen atom or a methyl group, particularly preferably a methyl group.

In $Y^{71}$, the $C_{1-10}$ alkylene group may, for example, be a linear alkylene group such as a methylene group, an ethylene group, a n-propylene group, a n-butylene group, a n-pentylene group, a n-hexylene group, a n-heptylene group, a n-octylene group, a n-nonylene group or a n-decylene group, or a branched alkylene group such as a 2-methylpropylene group, a 2-methylhexylene group or a tetramethylethylene group. Further, in the alkylene group, one or more hydrogen atoms may be substituted by a halogen atom. Among them, a $C_{1-10}$ linear alkylene group is preferred, and a methylene group, an ethylene group, a propylene group, a butylene group, a pentylene group or a hexylene group is more preferred.

The alkylene glycol residue means an alkyleneoxy group (-R-O-, wherein R is an alkylene group) which remains after the hydroxy group on one terminal or on both terminals of an alkylene glycol (HO-R-OH, wherein R is an alkylene group) is subjected to condensation reaction with other compound. For example, in the case of methylene glycol ($HO-CH_2-OH$), the alkylene glycol residue is a methyleneoxy group ($-CH_2-O-$), and in the case of ethylene glycol ($HO-CH_2CH_2-OH$), the alkylene glycol residue is an ethyleneoxy group ($-CH_2CH_2-O-$). Further, in the alkylene glycol residue, one or more hydrogen atoms may be substituted by a halogen atom.

The $C_{1-10}$ alkylene glycol residue as $Y^{71}$ may, for example, be a linear alkyleneoxy group such as a methyleneoxy group, an ethyleneoxy group, a n-propyleneoxy group, a n-butyleneoxy group, a n-pentyleneoxy group, a n-hexyleneoxy group, a n-heptyleneoxy group, a n-octyleneoxy group, a n-nonyleneoxy group or a n-decyleneoxy group, or a branched alkyleneoxy group such as a 2-methylpropyleneoxy group, a 2-methylhexyleneoxy group or a tetramethylethyleneoxy group. Among them, a $C_{1-10}$ linear alkyleneoxy group is preferred, a methyleneoxy group, an ethyleneoxy group, a propyleneoxy group, a butyleneoxy group, a pentyleneoxy group or a hexyleneoxy group is more preferred, and a methyleneoxy group, an ethyleneoxy group, a propyleneoxy group or a butyleneoxy group is further preferred.

The alkylene glycol residue itself has a property to suppress non-specific adsorption of protein. Accordingly, in a case where in the monomer (7), the linker $Y^{71}$ is an alkylene glycol residue, the units (a) derived from the monomer (7) have both property to fix the ligand and property to suppress non-specific adsorption of protein, etc. derived from cells.

p may be an integer of from 1 to 10. In a case where $Y^{71}$ is an alkylene glycol residue, p is preferably from 1 to 8, more preferably from 1 to 7, further preferably from 1 to 6.

In a case where in the specific resin composition, the units (a) contained in the polymer are constituted by a plural types of units differing in the number of p, p is specified as an average value in the entire resin composition. In a case where p is 2 or more, $Y^{71}$'s may be the same or different.

In a case where in the monomer (7), the linker $Y^{71}$ is an alkylene group, the total number of carbon atoms in the p alkylene groups ($(Y^{71})p$) is preferably from 1 to 100, more preferably from 1 to 20. In a case where p is 2 or more, $Y^{71}$'s may be the same or different.

The fixing group W is preferably a succinimide group or a thiol group, in view of high general purpose property with respect to the ligand to be fixed, as described above.

The monomer (7) may, for example, be more specifically a monomer represented by the following formula (8), a monomer represented by the following formula (9), a monomer represented by the following formula (10), a monomer represented by the following formula (11), a monomer represented by the following formula (17) or a monomer represented by the following formula (18):

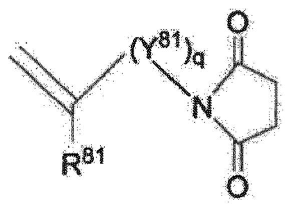 (8)

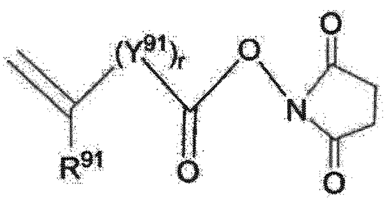 (9)

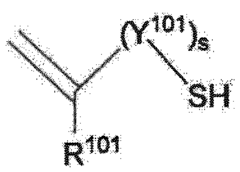 (10)

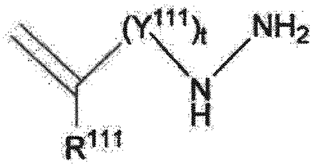 (11)

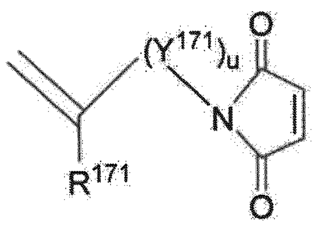 (17) 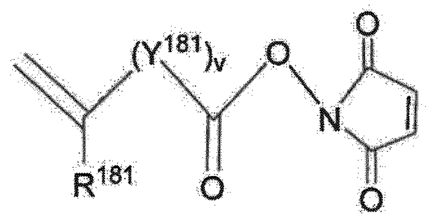 (18)

In the respective monomers, $Y^{81}$, $Y^{91}$, $Y^{101}$, $Y^{111}$, $Y^{171}$ and $Y^{181}$ are a single bond, -O-, -S-, -NH-, -CO-, -COO-, -CONH-, a $C_{1-10}$ alkylene group or a $C_{1-10}$ alkylene glycol group. q, r, s, t, u and v are an integer of from 1 to 10. A plurality of $Y^{81}$'s, $Y^{91}$'s, $Y^{101}$'s, $Y^{111}$'s, $Y^{171}$'s or $Y^{181}$'s (when q, r, s, t, u or v is 2 or more) present in one molecule of the monomer may be the same or different.

The $C_{1-10}$ alkylene glycol and the $C_{1-10}$ alkylene glycol residue as $Y^{81}$, $Y^{91}$, $Y^{101}$, $Y^{111}$, $Y^{171}$ and $Y^{181}$, may be as defined for the above-described $Y^{71}$.

In the respective monomers, the linkers $Y^{81}$, $Y^{91}$, $Y^{101}$, $Y^{111}$, $Y^{171}$ and $Y^{181}$ are preferably a single bond, a methylene group, an ethylene group or an ethyleneoxy group in view of favorable flexibility of the linker, and are more preferably a single bond, a methylene group or an ethyleneoxy group.

The numbers of repetition q, r, s, t, u and v may be respectively an integer of from 1 to 10.

In a case where $Y^{81}$, $Y^{91}$, $Y^{101}$, $Y^{111}$, $Y^{171}$ and $Y^{181}$ are an alkylene glycol residue, in the same manner as p for the above $Y^{71}$, q, r, s, t, u and v are preferably an integer of from 1 to 8, more preferably from 1 to 7, further preferably from 1 to 6. In a case where in the resin composition, the units (a) are constituted by a plural types of units differing in the number of q, r, s, t, u or v, q, r, s, t, u or v is specified as an average value in the entire resin composition. In a case where q, r, s, t, u and v are 2 or more, $Y^{81}$'s, $Y^{91}$'s, $Y^{101}$'s, $Y^{111}$'s, $Y^{171}$'s and $Y^{181}$'s may be the same or different.

In a case where the linker $Y^{81}$, $Y^{91}$, $Y^{101}$, $Y^{111}$, $Y^{171}$ or $Y^{181}$ is an alkylene group, in the same manner as in the case of the linker $Y^{71}$, the total number of carbon atoms in q, r, s, t, u or v alkylene groups is preferably from 1 to 100, more preferably from 1 to 20. In a case where q, r, s, t, u and v are 2 or more, $Y^{81}$'s, $Y^{91}$'s, $Y^{101}$'s, $Y^{111}$'s, $Y^{171}$'s and $Y^{181}$'s may be the same or different.

As the monomer (8), more specifically, a monomer represented by the following formula (8-1) or a monomer represented by the following formula (8-2) may, for example, be mentioned.

As the monomer (9), more specifically, a monomer represented by the following formula (9-1), a monomer represented by the following formula (9-2) or a monomer represented by the following formula (9-3) may, for example, be mentioned.

As the monomer (10), more specifically, a monomer represented by the following formula (10-1) or a monomer represented by the following formula (10-2) may, for example, be mentioned.

As the monomer (11), more specifically, a monomer represented by the following formula (11-1) or a monomer represented by the following formula (11-2) may, for example, be mentioned.

As the monomer (17), more specifically, a monomer represented by the following formula (17-1) or a monomer represented by the following formula (17-2) may, for example, be mentioned.

As the monomer (18), more specifically, a monomer represented by the following formula (18-1), a monomer represented by the following formula (18-2) or a monomer represented by the following formula (18-3) may, for example, be mentioned.

In the following monomers (8-1), (8-2), (9-2), (9-3), (10-1) to (11-2), (17-1), (17-2), (18-2) and (18-3), "a" is an integer of from 1 to 10, and "b" is an integer of from 1 to 6.

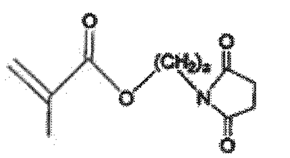 (8-1) 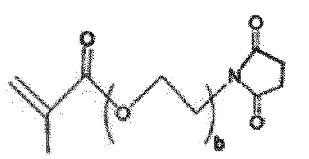 (8-2)

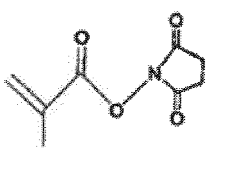 (9-1) 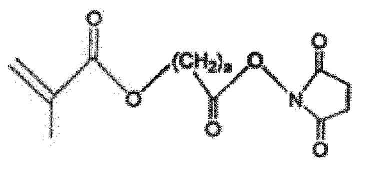 (9-2)

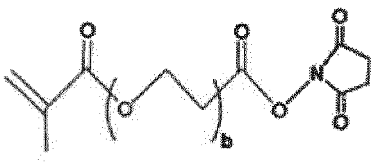 (9-3)

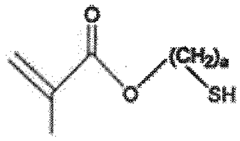 (10-1) 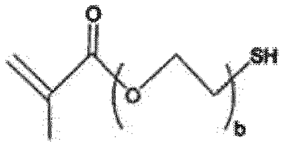 (10-2)

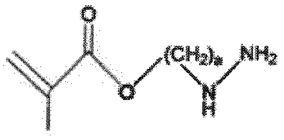 (11-1) 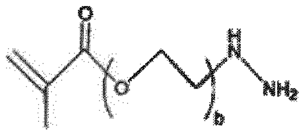 (11-2)

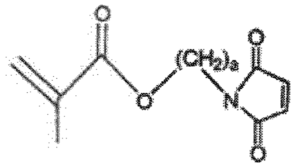 (17-1) 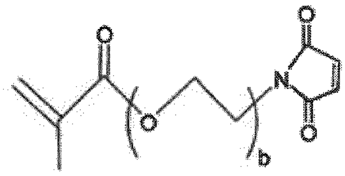 (17-2)

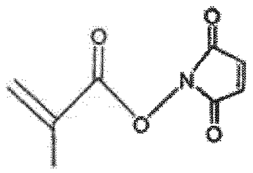 (18-1) 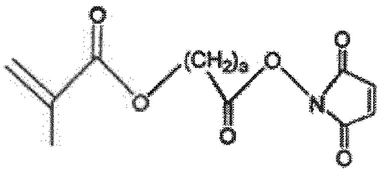 (18-2)

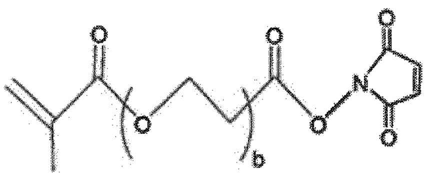 (18-3)

In a case where it is difficult to polymerize a monomer having a fixing group to obtain a composition, a monomer having its fixing group protected with a protecting group may be polymerized to obtain a polymer, and then the protecting group is removed.

In a case where the fixing group is a maleimide group or a succinimide group, the protecting group may, for example, be furan. In a case where the protecting group is furan, it can readily be removed by heating.

In a case where the fixing group is thiol, the protecting group may, for example, be a trimethylsilyl group, a triethylsilyl group or a tert-butyldimethylsilyl group. Such a group as a protecting group may readily be removed by an acid or a base in an aqueous solution.

In a case where the fixing group is a hydrazino group, the protecting group is not particularly limited so long as the amino group can be protected and may, for example, be a t-butoxycarbonyl group (Boc group), a benzyloxycarbonyl group (Z group, Cbz group) or a 9-fluorenylmethoxycarbonyl group (Fmoc group). The Boc group as a protecting group may readily be removed by a strong acid such as trifluoroacetic acid in an aqueous solution. Further, the Z group as a protecting group may readily be removed by blowing a hydrogen gas using as a catalyst e.g. palladium supported on activated carbon. Further, the Fmoc group as a protecting group may readily be removed by a tertiary amine such as pyrrolidine, piperidine or morpholine.

The proportion of the units (a) having a fixing group is not particularly limited and is, for example, preferably from 0.001 to 5 mol% to the total number of units constituting the composition, more preferably from 0.005 to 0.5 mol%, further preferably from 0.01 to 0.1 mol%. Here, the "total number of units constituting the composition" means the total

number of all the units which the polymer (the polymer having the units (a) and the units (b), the polymer (A) and the polymer (B)) contained in the specific resin composition has.

The proportion of the units (a) having a fixing group is not particularly limited and is, for example, preferably from 0.0004 to 3 mass%, more preferably from 0.002 to 0.6 mass%, further preferably from 0.004 to 0.3 mass% to the total number of units constituting the composition.

<<Unit (b)>>

In the specific resin composition, the units (b) have a biocompatible group. The specific resin composition, which has groups having biocompatibility, can suppress non-specific adsorption of protein, etc.

The biocompatible group is preferably at least one member selected from the group consisting of a group represented by the formula (1), a group represented by the formula (2) and a group represented by the formula (3), whereby a coating layer which has a high effect to suppress non-specific adsorption of protein can readily be formed. As the biocompatible group, preferred is the group (1) only, or one or both of the group (2) and the group (3) in that an effect to suppress non-specific adsorption of protein will readily be obtained, and particularly preferred is either one of the group (1), (2) or (3). The biocompatible group is particularly preferably the group (1) in view of availability.

Definitions and preferred embodiments of the respective symbols in the above formulae (1) to (3) are as defined above.

[Group (1)]

The group (1) can suppress non-specific adsorption of protein, etc. derived from cells to bind to the surface of the coating layer composed of the specific resin composition. The group (1) may be contained in the main chain of the unit (b) or may be contained in the side chain.

The group (1) may be linear or branched. The group (1) is preferably linear, in that a higher effect to suppress non-specific adsorption of protein, etc. derived from cells will be achieved. $R^{11}$ in the group (1) is preferably a methyl group or an ethyl group in view of excellent water resistance.

n in the group (1) is preferably from 1 to 10, particularly preferably from 1 to 5, in view of excellent water resistance, when the group (1) is contained in the side chain of the unit (b).

n in the group (1) is preferably from 2 to 10, particularly preferably from 4 to 10, in view of excellent water resistance, when the group (1) is contained in the main chain of the unit (b).

[Group (2)]

The group (2) has a strong affinity to phospholipids in the blood, etc., while its interaction force against plasma protein is weak. Accordingly, by the units (b) having the group (2), for example, it is considered that phospholipids are adsorbed preferentially on the coating layer made of the specific resin composition, and the phospholipids are self-assembled to form an adsorption layer. As a result, since the surface becomes a structure similar to the vascular endothelial surface, non-specific adsorption of proteins such as fibrinogen is suppressed.

The group (2) is contained in preferably in a side chain in the specific resin composition.

$R^{21}$ is a $C_{1-5}$ alkylene group and may, for example, be a linear alkylene group such as a methylene group, an ethylene group, a n-propylene group, a n-butylene group or a n-pentylene group, or a branched alkylene group such as a 2-methylpropylene group or a trimethylethylene group. Among them, in view of availability of the material, preferred is a $C_{1-5}$ linear alkylene group, more preferred is an ethylene group, a propylene group, a butylene group or a pentylene group, further preferred is an ethylene group.

$R^{22}$ is a $C_{1-5}$ alkylene group and may, for example, be the same group as $R^{21}$. Among them, with a view to suppressing non-specific adsorption of protein, preferred is a $C_{1-5}$ linear alkylene group, more preferred is a methylene group, an ethylene group, a propylene group or a butylene group, further preferred is an ethylene group.

$R^{23}$ to $R^{25}$ are each independently a $C_{1-5}$ alkyl group and may, for example, be a linear alkyl group such as a methyl group, an ethyl group, an n-propyl group, a n-butyl group or a n-pentyl group, or a branched alkyl group such as a 2-methylpropyl group or a trimethylethyl group. Among them, in view of availability of the material, preferred is a $C_{1-5}$ linear alkyl group, more preferred is a methyl group, an ethyl group, a propyl group or a butyl group, and further preferred is a methyl group.

In a case where the units (b) have the group (2), a single type of the group (2) may be contained, or two or more types may be contained.

[Group (3)]

By the units (b) having the group (3), non-specific adsorption of protein, etc. derived from cells can be suppressed from the same reasons as in the case where the units (b) have the group (2).

The group (3) is preferably contained in the side chain of the unit (b).

$R^{31}$ is a $C_{1-20}$ alkylene group and may, for example, be a linear alkyl group such as a methylene group, an ethylene group, a n-propylene group, a n-butylene group, a n-pentylene group, a n-hexylene group, a n-heptylene group, a n-octylene group, a n-nonylene group, a n-decylene group or a n-dodecylene group, or a branched alkylene group such as a 2-methylpropylene group, a 2-methylhexylene group or a tetramethylethylene group. In view of excellent flexibility of the specific resin composition, preferred is a $C_{1-20}$ linear alkylene group, more preferred is a methylene group, an ethylene group, a propylene group, a butylene group, a pentylene group, a hexylene group, a heptylene group, an octylene group, a nonylene group, a decylene group, a dodecylene group, a tridecylene group, a tetradecylene group or a pentadecylene group, further preferred is a methylene group, an ethylene group, a propylene group, a butylene group, a pentylene group, a hexylene group, a heptylene group, an octylene group, a nonylene group or a decylene group, and particularly preferred is an ethylene group.

$R^{34}$ is a $C_{1-5}$ alkylene group and may, for example, be the same group as $R^{21}$. Among them, with a view to suppressing non-specific adsorption of protein, preferred is a $C_{1-5}$ linear alkylene group, more preferred is a methylene group, an ethylene group, a propylene group or a butylene group, and further preferred is an ethylene group.

$R^{32}$ and $R^{33}$ are each independently a $C_{1-5}$ alkyl group and may, for example, be the same groups as $R^{23}$ to $R^{25}$. Among them, with a view to suppressing non-specific adsorption of protein, preferred is a $C_{1-5}$ linear alkyl group, more preferred is a methyl group, an ethyl group, a propyl group or a butyl group, and further preferred is a methyl group.

In a case where the units (b) have the group (3), a single type of the group (3) may be contained, or two or more types may be contained.

Further, in a case where the units (b) have the group (3), with a view to suppressing non-specific adsorption of protein, the units (b) preferably have either a group (3) wherein $X^-$ is the group (4) or a group (3) wherein $X^-$ is the group (5).

The monomer from which the units (b) having the biocompatible group are derived may, for example, be a monomer represented by the following formula (12):

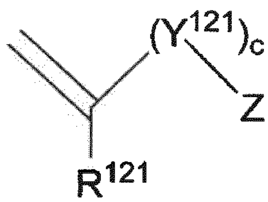

(12)

In the monomer (12), $R^{121}$ is a hydrogen atom, a halogen atom or a methyl group. $Y^{121}$ is a single bond, -O-, -S-, -NH-, -CO-, -COO-, -CONH-, a $C_{1-10}$ alkylene group or a $C_{1-10}$ alkylene glycol group. c is an integer of from 1 to 10. A plurality of $Y^{121}$'s present in one molecule of the monomer (12) (when c is 2 or more) may be the same or different. z is at least one group selected from the group consisting of the group (2) and the group (3).

In the monomer (12), $R^{121}$ is preferably a hydrogen atom or a methyl group, particularly preferably a methyl group.

The $C_{1-10}$ alkylene group in $Y^{121}$ may, for example, be the same group as $Y^{71}$. Further, in the alkylene group, a hydrogen atom may be substituted by a halogen atom. Preferred is a $C_{1-10}$ linear alkylene group, more preferred is a methylene group, an ethylene group, a propylene group, a butylene group, a pentylene group or a hexylene group.

The $C_{1-10}$ alkylene glycol residue as $Y^{121}$ may, for example, be the same residue as $Y^{71}$. Preferred is a $C_{1-10}$ linear alkyleneoxy group, more preferred is a methyleneoxy group, an ethyleneoxy group, a propyleneoxy group, a butyleneoxy group, a pentyleneoxy group or a hexyleneoxy group, further preferred is a methyleneoxy group, an ethyleneoxy group, a propyleneoxy group or a butyleneoxy group, particularly preferred is an ethyleneoxy group.

The number of repetition c of $Y^{121}$ may be an integer of from 1 to 10.

In a case where $Y^{121}$ is an alkylene glycol residue, c is preferably an integer of from 1 to 8, more preferably from 1 to 7, further preferably from 1 to 6. In a case where in the resin composition, the units (b) are constituted by a plural types of units differing in the number of c, c is specified as an average value in the entire resin composition. In a case where c is 2 or more, a plurality of $Y^{121}$'s may be the same or different.

In a case where in the monomer (12), the linker $Y^{121}$ is an alkylene group, the total number of carbon atoms in c alkylene groups (($Y^{121}$)c) is preferably from 1 to 100, more preferably from 1 to 20. In a case where c is 2 or more, a plurality of $Y^{121}$'s may be the same or different.

The biocompatible group Z is, as described above, preferably the group (1) only, or either one or both of the

group (2) and the group (3), in that an effect to suppress non-specific adsorption of protein will readily be obtained, particularly preferably either one of the group (1), the group (2) or the group (3).

The monomer (12) may, for example, be more specifically a monomer represented by the following formula (13), a monomer represented by the following formula (14) or a monomer represented by the following formula (15):

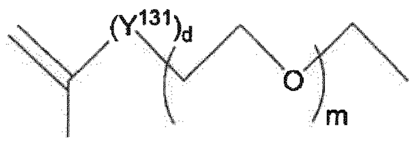

(13)

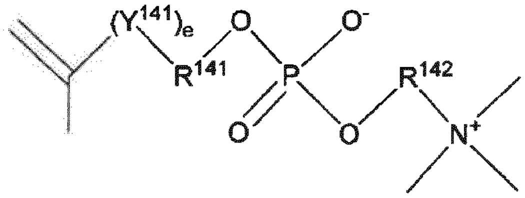

(14)

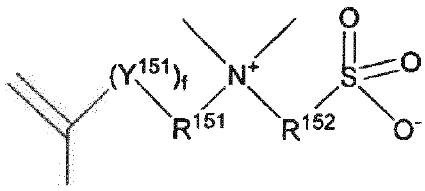

(15)

In the monomer, $Y^{131}$, $Y^{141}$ and $Y^{151}$ are the same ones as $Y^{101}$. d, e and f are an integer of from 1 to 10. A plurality of $Y^{131}$'s, $Y^{141}$'s or $Y^{151}$'s present in one molecule of the monomer (when d, e or f is 2 or more) may be the same or different. m is an integer of from 1 to 10. $R^{141}$, $R^{142}$ and $R^{152}$ are a $C_{1-5}$ alkylene group. $R^{151}$ is a $C_{1-20}$ alkylene group.

The $C_{1-10}$ alkylene group and the $C_{1-10}$ alkylene glycol residue as $Y^{131}$, $Y^{141}$ and $Y^{151}$ may, for example, be the same ones as $Y^{71}$.

In the respective monomers, the linkers $Y^{131}$, $Y^{141}$ and $Y^{151}$ are preferably a single bond, -O-, -CO-, -COO-, -CONH-, a methylene group, an ethylene group or an ethyleneoxy group, in view of excellent water resistance and in that the monomer is readily prepared, and more preferably a single bond, a methylene group or an ethyleneoxy group.

d, e and f may be an integer of from 1 to 10.

In a case where $Y^{131}$, $Y^{141}$ and $Y^{151}$ are an alkylene glycol residue, in the same manner as in the case of c in $Y^{121}$, d, e and f are preferably from 1 to 8, more preferably from 1 to 7, further preferably from 1 to 6. In the specific resin composition, in a case where the units (b) are constituted by a plural types of units differing in the number of d, e or f, d, e or f is specified as an average value in the entire resin composition. In a case where d, e and f are 2 or more, a plurality of $Y^{131}$'s, $Y^{141}$'s and $Y^{151}$'s may be the same or different.

In a case where the linkers $Y^{131}$, $Y^{141}$ and $Y^{151}$ are an alkylene group in the same manner as in the case of the linker $Y^{71}$, the total number of carbon atoms in d, e and f alkylene groups is preferably from 1 to 100, more preferably from 1 to 20. In a case where d, e and f are 2 or more, a plurality of $Y^{131}$'s, $Y^{141}$'s and $Y^{151}$'s may be the same or different.

As the monomer (13), the monomer (14) and the monomer (15), more specifically, a monomer represented by the following formula (13-1), a monomer represented by the following formula (14-1) and a monomer represented by the following formula (15-1) may, for example, be mentioned. In the monomer (13-1), k is an integer of from 1 to 10.

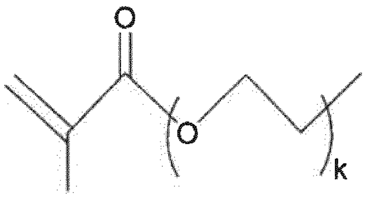

(13-1)

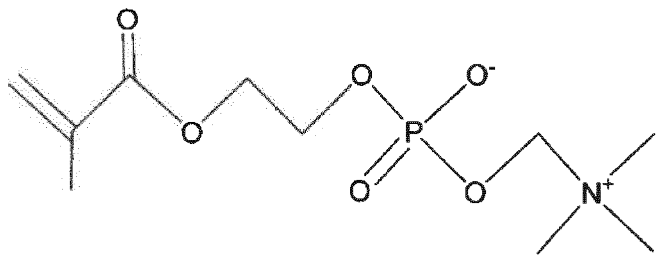

(14-1)

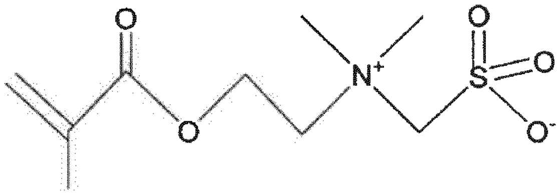

(15-1)

The proportion of the units (b) having a biocompatible group is not particularly limited and is, for example, preferably from 5 to 60 mass%, more preferably from 10 to 50 mass%, further preferably from 10 to 45 mass% to the total number of units constituting the composition.

<<Unit (c)>>

Further, in the specific resin composition, units (c) having a hydrophobic group may further be contained. By the presence of the units (c) in the specific resin composition, non-specific binding to hydrophilic substances is suppressed, and further, water resistance will improve.

[Group 6]

As the hydrophobic group, for example, a group represented by the following formula (6) may be mentioned:

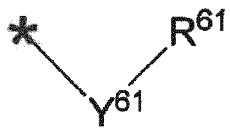

(6)

wherein * is a binding site, $Y^{61}$ is a single bond or a bivalent organic group, and $R^{61}$ is a $C_{1-20}$ alkyl group.

The group (6) may be contained in the main chain of the units (c) or may be contained in the side chain. The group (6) may be linear or branched.

As $Y^{61}$ in the group (6), in view of easiness of preparation, the following groups may be mentioned.

-O-, -S-, -NH-, $-SO_2-$, $-PO_2-$, -CH=CH-, -CH=N-, -N=N-, -N(O)=N-, -OCO-, -COO-, -COS-, -CONH-, $-COCH_2-$, $-CH_2CH_2-$, $-CH_2-$, $-CH_2NH-$, -CO-, -CH=CH-COO-, -CH=CH-CO-, a linear or branched alkylene group, an alkenylene group, an alkyleneoxy group, a bivalent 4- to 7-membered cyclic substituent, a bivalent 6-membered aromatic hydrocarbon group, a bivalent 4- to 6-membered alicyclic hydrocarbon group, a bivalent 5- or 6-membered heterocyclic group, a condensed ring thereof, a group constituted by a combination of bivalent linking groups, etc.

The bivalent organic group may have a substituent. The substituent may be a hydroxy group, a halogen atom, a cyano group, an alkoxy group (such as a methoxy group, an ethoxy group, a butoxy group, an octyloxy group or a methoxyethoxy group), an aryloxy group (such as a phenoxy group), an alkylthio group (such as a methylthio group or an ethylthio group), an acyl group (such as an acetyl group, a propionyl group or a benzoyl group), a sulfonyl group (such as a methanesulfonyl group or a benzenesulfonyl group), an acyloxy group (such as an acetoxy group or a benzoyloxy group), a sulfonyloxy group (such as a methanesulfonyloxy group or a toluenesulfonyloxy group), a phosphonyl group (such as a diethylphosphonyl group), an amide group (such as an acetylamino group or a benzoylamino group), a carbamoyl group (such as a N,N-dimethylcarbamoyl group or a N-phenylcarbamoyl group), an alkyl group (such as a methyl group, an ethyl group, a propyl group, an isopropyl group, a cyclopropyl group, a butyl group, a 2-carboxyethyl group or a benzyl group), an aryl group (such as a phenyl group or a tolyl group), a heterocyclic group (such as a pyridyl group, an imidazolyl group or a furanyl group), an alkenyl group (such as a vinyl group or a 1-propenyl

group), an alkoxyacyloxy group (such as an acetyloxy group or a benzoyloxy group), an alkoxycarbonyl group (such as a methoxycarbonyl group or an ethoxycarbonyl group), or a polymerizable group (such as a vinyl group, an acryloyl group, a methacryloyl group, a styryl group or a cinnamic acid residue).

$Y^{61}$ in the group (6) is preferably a single bond, -O-, $-(CH_2CH_2O)_\gamma-$ (wherein $\gamma$ is an integer of from 1 to 10), -COO-, a 6-membered aromatic hydrocarbon group, a linear or branched alkylene group, a linear or branched alkylene group in which one or more hydrogen atoms are substituted by a hydroxy group, or a group constituted by a combination of such bivalent linking groups, particularly preferably a single bond, a $C_{1-5}$ alkylene group, -COO- or $-COOA^1-$.

$A^1$ may be $-(CH_2)_\delta-$, $-(CH_2)_\delta-CH(OH)-(CH_2)_\varepsilon-$ or $-(CH_2)_\delta-NA^2-SO_2-$, and is particularly preferably $-(CH_2)_\delta-$, wherein $\delta$ is an integer of from 1 to 5, $\varepsilon$ is an integer of from 1 to 5, and $A^2$ is a hydrogen atom or a $C_{1-3}$ alkyl group.

$R^{61}$ in the group (6) is, in view of easiness of preparation, preferably a $C_{1-15}$ alkyl group, more preferably a $C_{1-12}$ alkyl group, further preferably a $C_{2-10}$ alkyl group.

As the monomer from which the units (c) having a hydrophobic group are derived, for example, a monomer represented by the following formula (16) may be mentioned.

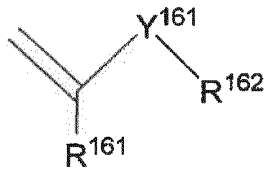

(16)

In the monomer (16), $Y^{161}$ is as defined for the above $Y^{61}$, $Y^{161}$ is a hydrogen atom, a halogen atom or a methyl group, and $R^{162}$ is as defined for the above $Y^{61}$.

$Y^{161}$ is a single bond, -O-, $-(CH_2CH_2O)_\gamma-$ (wherein $\gamma$ is an integer of from 1 to 10), -COO-, a 6-membered aromatic hydrocarbon group, a linear or branched alkylene group, a linear or branched alkylene group in which one or more hydrogen atoms are substituted by a hydroxy group, or a group constituted by a combination of such bivalent linking groups, particularly preferably a single bond, a $C_{1-5}$ alkylene group, -COO- or $-COOA^1-$.

$A^1$ may be $-(CH_2)_\delta-$, $-(CH_2)_\delta-CH(OH)-(CH_2)_\varepsilon-$ or $-(CH_2)_\delta-NA^2-SO_2-$, and is particularly preferably $-(CH_2)_\delta-$, wherein $\delta$ is an integer of from 1 to 5, $\varepsilon$ is an integer of from 1 to 5, and $A^2$ is a hydrogen atom or a $C_{1-3}$ alkylene group.

$R^{161}$ is preferably a halogen atom or a methyl group, particularly preferably a methyl group.

As the monomer (16), more specifically, the following monomers may be mentioned.

n-butyl(meth)acrylate, iso-butyl(meth)acrylate, sec-butyl(meth)acrylate, t-butyl (meth)acrylate, n-neopentyl(meth)acrylate, iso-neopentyl(meth)acrylate, iso-neopentyl (meth)acrylate, neopentyl(meth)acrylate, cyclohexyl(meth)acrylate, n-hexyl (meth)acrylate, iso-hexyl(meth)acrylate, heptyl(meth)acrylate, n-octyl(meth)acrylate, iso-octyl(meth)acrylate, 2-ethylhexyl(meth)acrylate, n-nonyl(meth)acrylate, iso-nonyl (meth)acrylate, n-decyl(meth)acrylate, iso-decyl(meth)acrylate, n-dodecyl (meth)acrylate, iso-dodecyl(meth)acrylate, n-tridecyl(meth)acrylate, iso-tridecyl (meth)acrylate, n-tetradecyl(meth)acrylate, iso-tetradecyl(meth)acrylate, n-pentadecyl (meth)acrylate, iso-pentadecyl(meth)acrylate, n-hexadecyl(meth)acrylate, iso-hexadecyl(meth)acrylate, n-octadecyl(meth)acrylate, iso-octadecyl(meth)acrylate, isobornyl(meth)acrylate, $CH_2=C(CH_3)COO(CH_2)_2(CF_2)_5CF_3$, $CH_2=CHCOO(CH_2)_2(CF_2)_5CF_3$, $CH_2=C(CH_3)COOCH_2CF_3$, $CH_2=CHCOOCH_2CF_3$, $CH_2=CR^6COO(CH_2)_eCF_2CF_2CF_3$, $CH_2=CR^6COO(CH_2)_eCF_2CF(CF_3)_2$, $CH_2=CR^6COOCH(CF_3)_2$, $CH_2=CR^6COOC(CF_3)_3$, etc.

The proportion of the units (c) having a hydrophobic group is not particularly limited and, for example, preferably from 0 to 90 mass%, more preferably from 30 to 80 mass%, further preferably from 50 to 80 mass% to the total number of units constituting the composition.

<<Unit (d)>>

Further, in the specific resin composition, units (d) having a crosslinkable group may be contained. The crosslinkable group can impart insolubility to the composition for example by crosslinking the main chains of the respective units. Further, it can make the resin composition and the substrate surface adhere to each other more firmly by crosslinking the unit to the solid phase surface of e.g. a substrate.

The crosslinking group is not particularly limited so long as it is a group crosslinking a unit to another unit or a group crosslinking a unit to the solid phase surface.

Such a crosslinking group may be introduced by polymerizing a monomer having a crosslinkable functional group and then reacting such crosslinkable functional groups to crosslink a unit to another unit.

The crosslinkable functional group is not particularly limited so long as it is a crosslinkable group unreactive during the polymerization of the monomer. It may, for example, be a functional group which forms a silanol group by hydrolysis, an epoxy group, a (meth)acrylic group or a glycidyl group.

The proportion of the units (d) is not particularly limited and for example, preferably from 0 to 10 mass%, more preferably from 0 to 5 mass%, further preferably from 0 to 2.5 mass% to the total number of units constituting the composition.

The resin composition of the present invention preferably contains from 0.002 to 3 mass% of the units (a), from 10 to 45 mass% of the units (b), from 50 to 80 mass% of the units (c) and from 0 to 2.5 mass% of the units (d).

<<Production method>>

The specific resin composition may be produced, for example, by a method of dissolving the monomers as the raw materials in an organic solvent, followed by polymerization to obtain a polymer. Disposition of the monomers in the polymer may be either random, block, graft or the like.

The organic solvent may, for example, be 2-butanone, ethanol, methanol, t-butyl alcohol, benzene, toluene, tetrahydrofuran, dioxane, dichloromethane, chloroform, acetone or methyl ethyl ketone.

The specific resin composition may be produced by dissolving the respective monomers in an organic solvent separately, polymerizing the monomers to produce homopolymers and mixing them, may be produced by producing two or more copolymers each obtained by copolymerizing two or more monomers and mixing the obtained two or more copolymers, or may be produced as a copolymer obtained by copolymerizing all material monomers.

That is, the specific resin composition may be produced by mixing the polymer (A) obtained by polymerizing a monomer from which the units (a) are derived and the polymer (B) obtained by polymerizing a monomer from which the units (b) are derived, or may be produced as a copolymer of a monomer from which the units (a) are derived and a monomer from which the units (b) are derived.

Further, as the specific resin composition, a fixing group may be introduced to the polymer (B) obtained by polymerizing a monomer from which the units (b) are derived to produce a copolymer having fixing groups and biocompatible groups. Further, in a case where the specific resin composition has the units (c), a monomer from which the units (b) are derived and a monomer from which the units (c) having a hydrophobic group are derived are polymerized to produce a copolymer having the units (b) and the units (c), and then some of the biocompatible groups or the hydrophobic groups in the copolymer are modified with a fixing group by a known chemical reaction to produce a copolymer also having the units (a) having a fixing group.

With a view to readily adjusting the proportion of the respective units, production by a method of polymerizing the raw material monomers for the respective units is preferred to the method of introducing fixing groups to the polymer. Particularly preferred is a method of copolymerizing two or more of the monomers to obtain a copolymer or a method of producing two or more copolymers by copolymerizing two or more of the monomers and mixing the obtained two or more copolymers. Further, in a case where the specific resin composition has the units (c), it is particularly preferred to mix a copolymer having the units (a) and the units (c) and a copolymer having the units (b) and the units (c) to obtain a resin composition.

With the specific resin composition, similar to another resin composition, the substrate surface may be coated to form a thin film, or a formed product can be produced.

<<Substrate>>

The present invention provides a substrate at least a part of which is coated with the specific resin composition.

According to the substrate of the present invention, it is possible to fix a more variety of ligands on the substrate surface. Further, the substrate having a ligand fixed may be used to selectively trap a target substance which specifically binds to the ligand. For example, the substrate having a ligand fixed may be used also as a column packing material for affinity chromatography to selectively trap a target substance.

The shape of the substrate is not particularly limited, and a plate shape, a spherical shape or the like may be mentioned. As the material of the substrate, for example, an inorganic substance may be silica, alumina, glass, a metal or the like. Further, an organic polymer material may be a thermoplastic resin or the like. More specifically, a linear polyolefin resin such as polyethylene or polypropylene; a cyclic polyolefin resin; or a fluorinated resin may, for example, be mentioned.

The saturated cyclic polyolefin resin may be a homopolymer having a cyclic olefin structure or a saturated polymer obtained by hydrogenating a copolymer of a cyclic olefin and an $\alpha$-olefin.

Further, in a case where the substrate is for cell culture, its material is not particularly limited so long as it is an optional material suitable for incubating cells, and may, for example, be a glass material such as soda lime glass, PYREX (tradename) glass, Vycor (tradename) glass or quartz glass; silicon; a plastic containing a dendritic polymer or polymer, such as poly(vinyl chloride), poly(vinyl alcohol), poly(methyl methacrylate), poly(vinyl acetate/maleic anhydride), poly(dimethylsiloxane) monomethacrylate, a cyclic olefin polymer, a fluorocarbon polymer, a polystyrene, a polypropylene or a polyethyleneimine; a copolymer such as poly(vinyl acetate/maleic anhydride), poly(styrene/maleic anhydride), poly(ethylene/acrylic acid) or a derivative thereof.

As the substrate, more specifically, a carrier (such as a magnetic carrier or a carrier for affinity column purification), a substrate for cell culture, a preparation, a microdevice or a membrane may, for example, be mentioned. As the substrate for cell culture, a multi-well plate having an optional number of wells disposed or a petri dish may, for example, be mentioned. The number of wells may, for example, be 6, 12, 24, 94, 384 or 1,536 per plate.

Further, in a case where the substrate is spherical particles, preferred are polymer particles having an average particle size of from 0.1 to 500 μm. Carrier particles having a particle size within the above range are considered to be readily recovered by e.g. centrifugal separation or a filter, and have a sufficient surface area and thereby have a high reaction efficiency with a target substance. When the average particle size is at most 500 μm, the surface area is not too small, and the reaction efficiency with protein is high. When the average particle size is at least 0.1 μm, the particles can be efficiently recovered by a filter, and in a case where the particles are used as packed in a column, the pressure loss at the time of liquid flow will not be significant.

<<Coating method>>

To coat the substrate surface with the specific resin composition, for example, a solution having the polymer compound dissolved in an organic solvent is applied to the substrate e.g. by dipping, spraying or spin coating, followed by drying in an environment at from about 10 to about 120°C. As the organic solvent, those the same as described in the above <<Production method>> may be mentioned.

The thickness of the coating layer is preferably from 1 nm to 1 mm, particularly preferably from 5 nm to 800 μm. When the thickness is at least the above lower limit value, non-specific adsorption of e.g. unnecessary protein derived from e.g. cells can be suppressed. When the thickness is at most the above upper limit value, the coating layer is likely to adhere to the substrate surface.

<<Ligand fixation method>>

By fixing the ligand to the substrate the surface of which is coated with the specific resin composition, a substrate capable of selectively trapping a target substance can be produced. The method of fixing the ligand may be determined by those skilled in the art in accordance with a known method depending upon the fixing group which the coating layer on the substrate has by a covalent bond. For example, a method of bringing a solution containing a ligand into contact with a substrate having a coating layer having a fixing group capable of being covalently bonded to the ligand may, for example, be mentioned.

In a case where the fixing group on the substrate surface is a succinimide group and the ligand having an amino group is to be fixed, for example, by incubation for a predetermined time in a state where the substrate surface is in contact with a solution having the ligand mixed in a conventional buffer solution having a pH of from 7.0 to 10.0, the ligand can be bonded to the fixing group. The buffer solution may, for example, be a phosphate buffer solution or a tris buffer solution.

In a case where the substrate is for cell culture, the ligand to be fixed is not particularly limited so long as it is a substance which specifically binds to the surface of the target cells and may, for example, be an antibody, an antibody fragment, an aptamer or a cell adhesion factor.

The antibody may be prepared, for example, by immunizing a rodent such as a mouse with a labeled peptide as an antigen. Further, it may be prepared by screening a phage library. The antibody fragment may, for example, be Fv, Fab or scFv.

The aptamer is a substance which has a specific binding ability to an aimed substance. The aptamer may, for example, be a peptide aptamer. The peptide aptamer which has a specific binding ability to a target substance may be selected, for example, by two-hybrid screening employing yeast.

The cell adhesion factor is a general name for molecules which have a roll in cell adhesion. The cell adhesion factor may, for example, be fibronectin, laminin, fibrinogen or thrombospondin and is not limited thereto. The cell adhesion factor may be properly selected depending upon the cells to be incubated.

A natural cell adhesion factor may be directly obtained by a known recovery method and purification method from a natural product, or may be obtained in such a manner that by a known gene recombination technique, a gene encoding the protein is integrated into an expression vector and is thereby introduced into a cell to express the cell adhesion factor, which is then recovered and purified by a known method. Otherwise, the protein may be produced by means of cell-free protein synthesis system employing a commercial kit such as agent kit PROTEIOS™ (TOYOBO CO., LTD.), TNTTM System (Promega K.K.), a synthesis apparatus PG-Mate™ (TOYOBO CO., LTD.) or RTS (Roche Diagnostics K.K.) and recovered and purified by a known method.

Further, a chemically synthesized cell adhesion factor may be obtained by a known protein synthesis method. The synthesis method may, for example, be azide method, acid chloride method, acid anhydride method, mixed anhydride

method, DCC method, active ester method, carboimidazole method or oxidation-reduction method. Further, for synthesis, either solid phase synthesis or solution phase synthesis may be employed. A commercial protein synthesis apparatus may be used. After the synthesis reaction, the cell adhesion factor may be purified employing known purification methods such as chromatography in combination.

The target substance which binds to the ligand is not particularly limited and may, for example, be an antigen, an antibody, a chemical agent (a synthetic compound or a natural compound), a nucleic acid or a cell.

Since the ligand and the fixing group are covalently bonded, even when the target substance is to be bonded to the substrate, the ligand is stably fixed to the substrate and will not improperly be liberated.

<<Treatment to inactivate fixing group>>

After fixation of the ligand, after the solution for the reaction between the ligand and the fixing group is removed, it is preferred to carry out a treatment to inactivate the fixing group which was not involved in fixation of the ligand. The inactivation treatment may be carried out by converting the fixing group into other group which will not bind to the protein, depending upon the type of the fixing group. For example, in a case where the fixing group is a maleimide group or a succinimide group, it may be inactivated with a reducing agent such as mercaptoethanol. In a case where the fixing group is a thiol group, it may be inactivated with iodoacetic acid, N-ethylmaleimide or the like. In a case where the fixing group is a hydrazino group, it may be inactivated with an acid anhydride such acetic anhydride or succinic anhydride.

<Cell culture method>

The present invention provides a cell culture process, which comprises a step of bonding a ligand having a moiety which specifically binds to the surface of target cells, to at least one functional group selected from the group consisting of a maleimide group, a succinimide group, a thiol group and a hydrazino group on the surface of the substrate, a step of bringing the target cells into contact with the substrate having the ligand bonded thereto, to bond the target cells to the ligand, and a step of incubating the target cells bonded to the ligand.

According to the cell culture process of the present invention, by bringing a biological sample containing target cells which specifically bind to the fixed ligand, into contact with the substrate surface, only the target cells can be selectively trapped and purely cultivated. Further, by properly selecting the type of the ligand to be fixed, a variety of cells can be cultivated.

<<Ligand bonding step>>

The ligand can be fixed to the substrate surface in the same manner as the above-described <<Ligand fixation method>>. Further, as the ligand, the same ligands as those described above may be used.

<<Target cell bonding step>>

Then, a biological sample containing target cells is brought into contact with the substrate having the ligand fixed thereto so that the target cells are bonded to the ligand on the substrate surface and selectively trapped. Bonding of the ligand and the target cells may be reversible or irreversible.

The biological sample is not particularly limited and may, for example, be a suspension of cells prepared from body fluids such as the blood, the blood plasma, the blood serum, the lymph, the saliva, tears, the urine or the sweat, or a tissue fragment. The biological sample to be brought into contact with the substrate may be subjected to a pretreatment such that a sample collected from a living organism is diluted with e.g. a buffer solution.

The time over which the target cells and the substrate are brought into contact with each other is from 1 to 24 hours.

<<Incubation step>>

Then, a medium in a sufficient amount is added to incubate the target cells. The incubation time may be properly set depending upon the type of the cells. Before incubation, substances other than the target cells, for example, protein, dead cells, or cells other than the target cells, contained in the biological sample, may be washed away by using a medium.

The medium used may be any basal medium containing components necessary for survival and proliferation of cells (inorganic salts, carbohydrates, hormones, essential amino acids, non-essential amino acids, vitamins) and is properly selected depending upon the type of the cells. For example, DMEM, Minimum Essential Medium (MEM), RPMI-1640, Basal Medium Eagle (BME), Dulbecco's Modified Eagle's Medium: Nutrient Mixture F-12 (DMEM/F-12) and Glasgow Minimum Essential Medium (Glasgow MEM) may be mentioned.

EXAMPLES

Now, the present invention will be described in further detail with reference to Examples. However, it should be understood that the present invention is by no means restricted to the following Examples.

[Production Example 1]

(1) Preparation of monomer from which units having fixing group are derived

Into a 1 L (liter) three-necked flask, 45.2 g (160 mmol) of hexaethylene glycol, 7.63 g (40 mmol) of p-toluenesulfonyl chloride and 400 ml of chloroform were added. Then, in the obtained mixture, a mixture of 5.70 g (56 mmol) of triethylamine and 100 mL of chloroform was dropped at 0°C in a nitrogen atmosphere, followed by stirring at room temperature for 16 hours. Then, the obtained reaction mixture was put in a 1 L separatory funnel, and the organic layer was washed with a 1 N aqueous hydrochloric acid solution once and with a saturated salt solution twice. Then, the obtained organic layer was dried over sodium sulfate and concentrated and then purified by silica gel column chromatography using ethyl acetate:methanol = 9:1 (vol) as a developing solvent, whereupon a colorless transparent liquid (the following compound A) was obtained in an amount of 13.0 g with a yield of 74.7%.

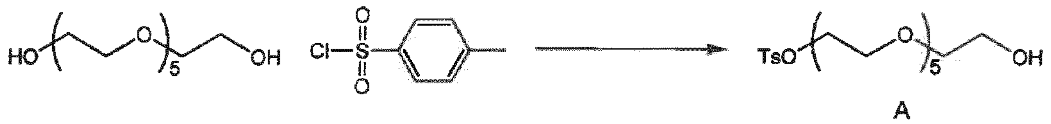

A

Then, into a 1 L two-necked flask, 29.1 g (300 mmol) of maleimide, 61.3 g (900 mmol) of furan, 22 mg (0.1 mmol) of dibutylhydroxytoluene and 600 mL of toluene were added. The obtained mixture was stirred at 60°C in a nitrogen atmosphere for 24 hours, the resulting reaction liquid was cooled with ice, and precipitated crystals were collected by filtration and washed with cold toluene, whereupon a white powder (the following compound B) was obtained in an amount of 43.5 g with a yield of 87.8%.

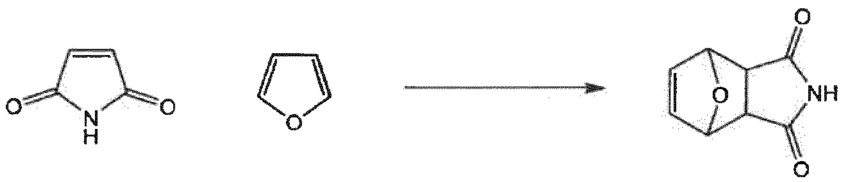

B

Then, into a 500 mL two-necked flask, 13.0 g (30 mmol) of compound A, 7.43 g (45 mmol) of compound B, 20.7 g (150 mmol) of potassium carbonate and 300 mL of acetonitrile were added. The obtained mixture was stirred under reflux in a nitrogen atmosphere for 2 hours. The obtained reaction mixture was put in a 1 L separatory funnel, 500 mL of chloroform was added, and the organic layer was washed with a 1 N aqueous hydrochloric acid solution once and with a saturated salt solution twice. The obtained organic layer was dried over sodium sulfate and concentrated, and then purified by silica gel column chromatography using ethyl acetate:methanol = 8:2 (vol) as a developing solvent, whereupon a colorless transparent liquid (the following compound C) was obtained in an amount of 6.29 g with a yield of 48.8%.

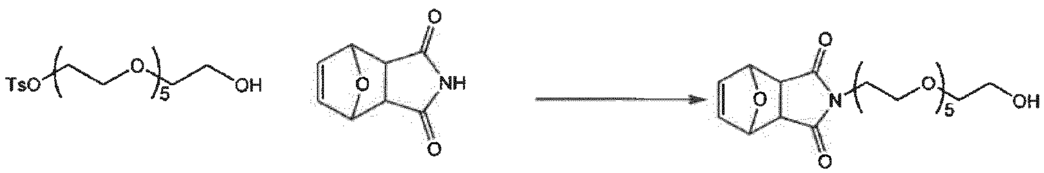

A B C

The obtained compound C was subjected to $^1$H-NMR analysis, and the results are as follows.

$^1$H-NMR (300 MHz, $CDCl_3$)$\delta$=6.52(2H, CH=CH), 5.26(2H, CHOCH), 3.69-3.60(2H+20H+2H, $NCH_2$, $CH_2OCH_2$, $CH_2CH_2OH$), 2.87(2H, CHCHCON).

Then, into a 300 mL three-necked flask, 6.01 g (14 mmol) of compound C, 2.83 g (28 mmol) of triethylamine and 100 mL of chloroform were added. Then, in the obtained mixture, a mixture of 1.76 g (16.8 mmol) of methacrylic acid chloride and 50 mL of chloroform was dropped at 0°C in a nitrogen atmosphere, followed by stirring at room temperature for one hour. Then, the obtained reaction mixture was put in a 500 mL separatory funnel, and the organic layer was washed with a 1 N aqueous hydrochloric acid solution once and with a saturated salt solution twice. The obtained organic layer was dried over sodium sulfate and concentrated, and then purified by silica gel column chromatography using ethyl acetate: methanol = 9:1 (vol) as a developing solvent, whereupon a colorless transparent liquid (the following compound D) was obtained in an amount of 4.86 g with a yield of 69.8%.

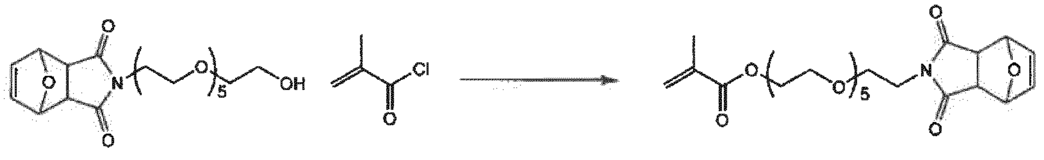

C D

The obtained compound D was subjected to $^1$H-NMR, and the results are as follows.
$^1$H-NMR (300 MHz, $CDCl_3$)δ=6.51(2H, CH=CH), 6.13(1H, $C(CH_3)CH_2$), 5.58(1H, $C(CH_3)CH_2$), 5.26(2H, CHOCH), 4.30(2H, $COOCH_2$), 3.76-3.60(20H+2H, $CH_2OCH_2$, $CH_2N$), 2.86(2H, NCOCHCH), 1.95(3H, $CCH_3$).

(2) Preparation of polymer having units having hydrophobic group and units having fixing group

In a 100 mL three-necked flask, 1.80 g (18 mmol) of methyl methacrylate, 0.995 g (2 mmol) of the compound D, 28.0 mg (0.113 mmol) of 2,2'-azobis(2,4-dimethylvaleronitrile) and 11.2 g of toluene were added. Then, the concentration of monomers in the reaction liquid was adjusted to be 20 mass% and the initiator concentration was adjusted to be 1 mass%. Then, the obtained mixture was stirred at 58°C in a nitrogen atmosphere for 16 hours, and the resulting reaction liquid was cooled with ice and dropped in hexane to precipitate a polymer, The obtained polymer was sufficiently washed with hexane and vacuum dried to obtain a white powdery polymer E in an amount of 1.47 g with a yield of 52.5%.

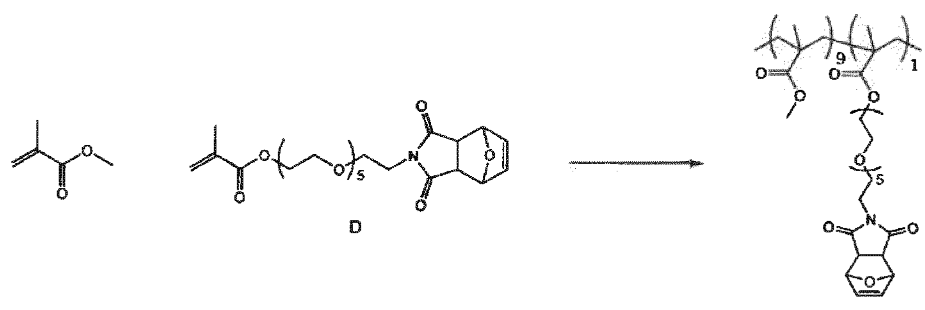

D E

Then, to let furan as a protecting group for the maleimide group leave, in a 100 mL flask, 1.0 g of the polymer E, 1 mg (4.5 μmol) of dibutylhydroxytoluene and 20 g of toluene were added. The obtained mixture was stirred under reflux for 3 hours, cooled with ice and dropped in hexane to precipitate a polymer. The obtained polymer was sufficiently washed with hexane and vacuum dried to obtain 0.76 g of a white powdery polymer F.

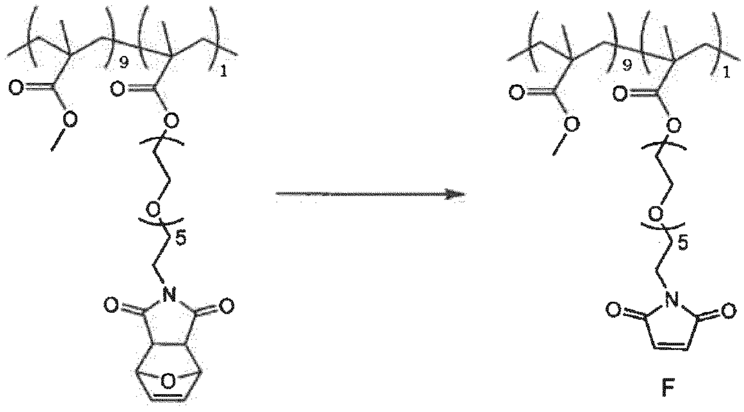

E F

[Production Example 2]

(1) Preparation of polymer having units having hydrophobic group and units having biocompatible group

Into a 100 mL three-necked flask, 4.93 g (20 mmol) of polyethylene glycol monoethyl ether monomethacrylate, 2.00 g (20 mmol) of methyl methacrylate, 69.3 mg (0.279 mmol) of 2,2'-azobis(2,4-dimethylvaleronitrile) and 27.7 g of toluene were added. Then, the concentration of monomers in the reaction liquid was adjusted to be 20 mass%, and the initiator concentration was adjusted to be 1 mass%. Then, the obtained mixture was stirred at 58°C in a nitrogen atmosphere for 16 hours, and the resulting reaction liquid was cooled with ice and dropped in hexane to precipitate a polymer. The obtained polymer was sufficiently washed with hexane and vacuum dried to obtain a white powdery polymer G.

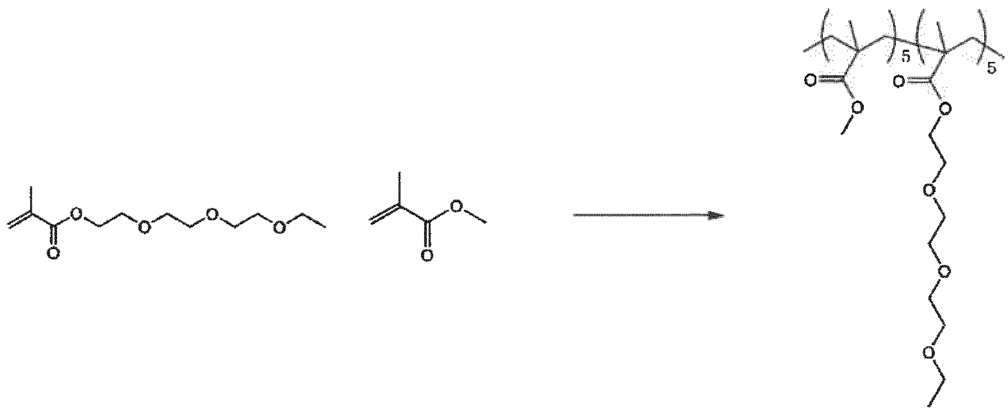

G

[Production Example 3]

(1) Preparation of monomer from which units having fixing group are derived

In the same manner as in Production Example 1 except that 1,3-propanediol was used instead of hexaethylene glycol, a monomer from which units having a fixing group are derived was prepared to obtain a compound H.

(2) Preparation of polymer having units having hydrophobic group and units having fixing group

Into a 100 mL three-necked flask, 2.70 g (27 mmol) of methyl methacrylate, 0.87 g (3 mmol) of the compound H, 35.7 mg (0.144 mmol) of 2,2'-azobis(2,4-dimethylvaleronitrile) and 14.3 g of toluene were added. Then, the concentration of monomers in the reaction liquid was adjusted to be 20 mass% and the initiator concentration was adjusted to be 1 mass%. Then, the obtained mixture was stirred at 58°C in a nitrogen atmosphere for 16 hours, and the resulting reaction liquid was cooled with ice and dropped in hexane to precipitate a polymer. The obtained polymer was sufficiently washed with hexane and vacuum dried to obtain a white powdery polymer I in an amount of 1.53 g with a yield of 42.9%.

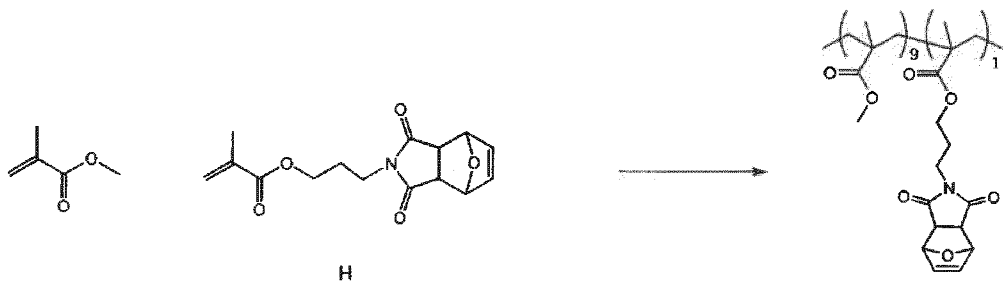

H

I

Then, in order to let furan as a protecting group for the maleimide group leave, in a 100 mL flask, 1.0 g of the polymer I, 1 mg (4.5 μmol) of dibutylhydroxytoluene and 20 g of toluene were added. The obtained mixture was stirred under reflux for 3 hours, cooled with ice and dropped with hexane to precipitate a polymer. The obtained polymer was sufficiently washed with hexane and vacuum dried to obtain 0.82 g of a white powdery polymer J.

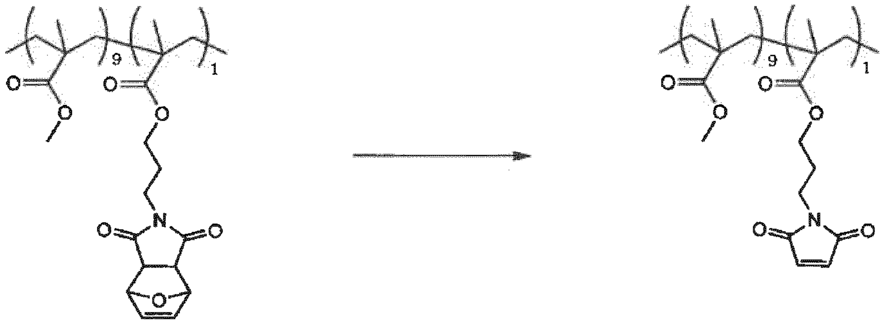

I J

[Production Example 4]

(1) Preparation of monomer from which units having fixing group are derived

In the same manner as in Production Example 1 except that 1,6-hexanediol was used instead of hexaethylene glycol, a monomer from which units having a fixing group are derived was prepared to obtain a compound K.

(2) Preparation of polymer having units having hydrophobic group and units having fixing group

Into a 100 mL three-necked flask, 3.60 g (36 mmol) of methyl methacrylate, 1.33 g (4 mmol) of the compound K, 49.3 mg (0.198 mmol) of 2,2'-azobis(2,4-dimethylvaleronitrile) and 19.7 g of toluene were added. Then, the concentration of monomers in the reaction liquid was adjusted to be 20 mass% and the initiator concentration was adjusted to be 1 mass%. The obtained mixture was stirred at 58°C in a nitrogen atmosphere for 16 hours, and the resulting reaction liquid was cooled with ice and dropped in hexane to precipitate a polymer. The obtained polymer was sufficiently washed with hexane and vacuum dried to obtain a white powdery polymer L in an amount of 0.68 g with a yield of 13.8%.

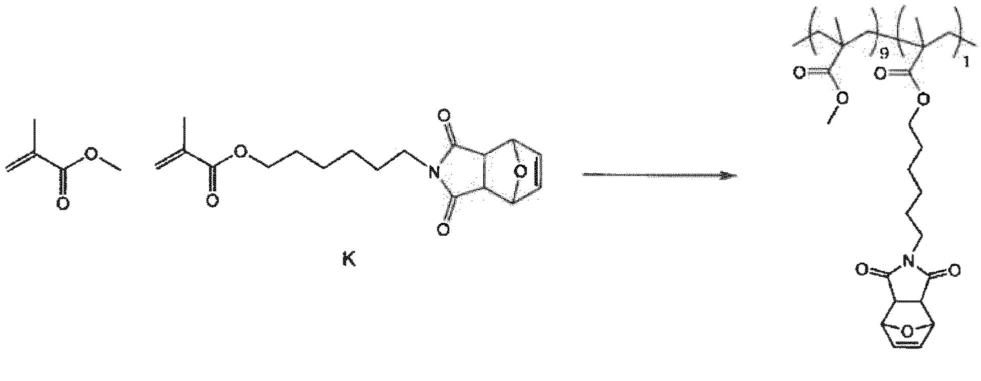

K

L

Then, in order to let furan as a protecting group for the maleimide group leave, in a 100 mL flask, 0.5 g of the polymer L, 1 mg (4.5 μmol) of dibutylhydroxytoluene and 10 g of toluene were added. The obtained mixture was stirred under reflux for 3 hours, cooled with ice and dropped in hexane to precipitate a polymer. The obtained polymer was sufficiently washed with hexane and vacuum dried to obtain 0.37 g of a white powdery polymer M.

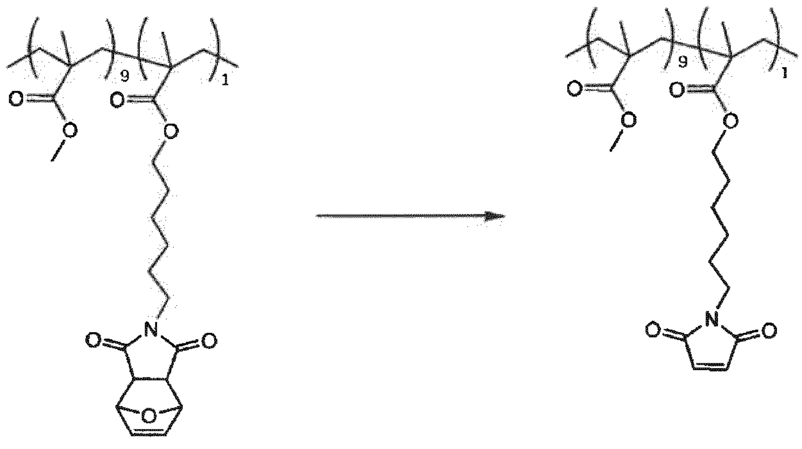

L M

[Ex. 1]

The polymer F obtained in Production Example 1 and the polymer G obtained in Production Example 2 were weighed so that their weight ratio would be 100:0, 99.5:0.5, 99.0:1.0, 95.0:5.0, 90.0:10.0, 50:50 and 0:100 as identified in Table 2, and dissolved in AK-225 (manufactured by Asahi Glass Company, Limited) so that their concentration would be 0.1 mass% to prepare coating solutions. Then, each coating solution was dispensed to 3 wells of a 24-well microplate (manufactured by ATG) in an amount of 2.2 mL per well and left to stand for one day to volatilize the solvent thereby to form a coating layer on the well surface.

[Ex. 2]

Coating solutions were prepared in the same manner as in Ex. 1 except that the polymer J obtained in Production Example 3 was used instead of the polymer (F) and the weight ratio of the polymer J and the polymer G obtained in Production Example 2 would be 100:0, 99.5:0.5, 99.0:1.0, 95.0:5.0, 90.0:10.0 and 50:50 as identified in Table 2. Then, using each coating solution, in the same manner as in Ex. 1, a coating layer was formed on the well surface of a 24-well microplate.

[Ex. 3]

Coating solutions were prepared in the same manner as in Ex. 1 except that the polymer M obtained in Production Example 4 was used instead of the polymer (F) and the weight ratio of the polymer M and the polymer G obtained in Production Example 2 would be 100:0, 99.5:0.5, 99.0:1.0, 95.0:5.0, 90.0:10.0 and 50:50 as identified in Table 2. Then,

using each coating solution, in the same manner as in Ex. 1, a coating layer was formed on the well surface of a 24-well microplate.

[Test Example 1] Test on water-insolubility of polymer

10 mg of each of the polymers F, J and M obtained in Production Examples 1, 3 and 4 and 1 g of water were weighed into a sample tube and stirred at room temperature for one hour, whereupon the water-insolubility was visually confirmed. The evaluation was carried out on the basis of the following standards. The results are shown in Table 1.

<Evaluation standards>

○ (good): The fluoropolymer remained.
× (bad): The fluoropolymer was completely dissolved and did not remain.

[Table 1]

| Type of polymer | Evaluation results |
|---|---|
| Polymer F | ○ |
| Polymer J | ○ |
| Polymer M | ○ |

From Table 1, each polymer was confirmed to have sufficient water-insolubility.

[Test Example 2] Test on confirmation of protein non-adsorption of microplate

(1) Preparation of coloring solution and protein solution

As the coloring solution, one having 50 mL of a peroxidase color solution (3,3',5,5'-tetramethy)benzidine (TMBZ), manufactured by KPL, Inc.) and 50 mL of TMB Peroxidase Substrate (manufactured by KPL, Inc.) mixed, was used. As the protein solution, one having protein (POD-goat anti mouse IgG, manufactured by Bio-Rad Laboratories, Inc.) diluted 16,000-fold with phosphate buffer solution (D-PBS, manufactured by Sigma Co.), was used.

(2) Protein adsorption

To the wells each having the coating layer formed thereon of the 24-well microplate having the coating layer formed obtained in each of Ex. 1 to 3, 2 mL of the protein solution was dispensed (2 mL per well) and left to stand at room temperature for one hour. As a blank, the protein solution was dispensed to 3 wells of a non-coated 96-well microplate in an amount of 2 μL (2 μL per well).

(3) Washing of wells

Then, the 24-well microplate was washed four times with 4 mL of phosphate buffer solution (D-PBS, manufactured by Sigma Co.) having 0.05 mass% of a surfactant (Tween 20, manufactured by Wako Pure Chemical Industries, Ltd.) incorporated (using 4 mL per well).

(4) Dispensing of coloring solution

Then, to the washed 24-well microplate, 2 mL of the coloring solution was dispensed (using 2 mL per well), and a coloring reaction was carried out for 7 minutes. The coloring reaction was stopped by adding 1 mL of 2N sulfuric acid (using 1 mL per well). As the blank, to the 96-well microplate, 100 μL of the coloring solution was dispensed (using 100 μL per well), and a coloring reaction was carried out for 7 minutes. The coloring reaction was stopped by adding 50 μL of 2N sulfuric acid (using 50 μL per well).

(5) Measurement of absorbance and calculation of protein adsorption rate Q

Then, from each well of the 24-well microplate, 150 μL of the liquid was taken and transferred to the 96-well microplate. As to the absorbance, the absorbance at 450 nm was measured by MTP-810Lab (manufactured by Corona Electric Co., Ltd.). Here, the average value of the absorbance (N=3) of the blank was designated as $A_0$. The absorbance of the liquid transferred from the 24-well microplate to the 96-well microplates was designated as $A_1$. The protein adsorption rate $Q_1$ was obtained by the following formula, and the protein adsorption rate Q was set to be the average value. The results are shown in Table 2. Q is preferably at most 0.2%, more preferably at most 0.1%.

$$Q_1 = A_1/\{A_0\times(100/\text{dispensed amount of the protein solution in the blank})\}\times 100 = A_1/\{A_0\times(100/2\ \mu L)\}\times 100\ [\%]$$

[Table 2]

| | | Polymer G | Polymer F | Polymer J | Polymer M | Protein adsorption rate (%) |
|---|---|---|---|---|---|---|
| Ex. 1 | Ex. 1-1 | 100 | 0 | 0 | 0 | 0.029 |
| | Ex. 1-2 | 99.5 | 0.5 | 0 | 0 | 0.044 |
| | Ex. 1-3 | 99 | 1 | 0 | 0 | 0.022 |
| | Ex. 1-4 | 95 | 5 | 0 | 0 | 0.045 |
| | Ex. 1-5 | 90 | 10 | 0 | 0 | 0.027 |
| | Ex. 1-6 | 50 | 50 | 0 | 0 | 0.052 |
| | Ex. 1-7 | 0 | 100 | 0 | 0 | 0.324 |
| Ex. 2 | Ex. 2-1 | 99.5 | 0 | 0.5 | 0 | 0.038 |
| | Ex. 2-2 | 99 | 0 | 1 | 0 | 0.021 |
| | Ex. 2-3 | 95 | 0 | 5 | 0 | 0.037 |
| | Ex. 2-4 | 90 | 0 | 10 | 0 | 0.019 |
| | Ex. 2-5 | 50 | 0 | 50 | 0 | 0.060 |
| | Ex. 2-6 | 0 | 0 | 100 | 0 | 1.185 |
| Ex. 3 | Ex. 3-1 | 99.5 | 0 | 0 | 0.5 | 0.035 |
| | Ex. 3-2 | 99 | 0 | 0 | 1 | 0.036 |
| | Ex. 3-3 | 95 | 0 | 0 | 5 | 0.036 |
| | Ex. 3-4 | 90 | 0 | 0 | 10 | 0.038 |
| | Ex. 3-5 | 50 | 0 | 0 | 50 | 0.070 |
| | Ex. 3-6 | 0 | 0 | 0 | 100 | 1.247 |

(6) Results

In Table 2, Ex. 1-1, 1-7, 2-6 and 3-6 are Comparative Examples. Ex. 1-2 to 1-6, 2-1 to 2-5 and 3-1 to 3-5 are Examples of the present invention.

In Table 2, it was confirmed that in Ex. 1 to 3, by the units having a group having biocompatibility, non-specific adsorption of protein is suppressed. It is found that in Ex. 1-7, 2-6 and 3-6, the protein adsorption rate is higher than 0.2% and non-specific adsorption occurred. Further, from the results in Ex. 1-7, with the polymer in Ex. 1, non-specific adsorption of protein tends to be suppressed since the linker of the polymer F is a polyethylene glycol group, however, suppression is insufficient as compared with other Examples of the present invention.

[Test Example 3] Test on confirmation of peptide selective fixation of plate

(1) Preparation of plate coated with polymer

Among the 24-well microplates having the coating layer formed thereon obtained in Ex. 1 to 3, wells coated with polymers in a proportion in Ex. 1-5, 2-4 and 3-4 in Table 2 were employed.

(2) Fixation of peptide

Then, an RGD peptide (containing cysteine) having cell adhesion activity was dispensed in an amount of 0.1 μmol per 1 well so as to be reacted with maleimide in the well thereby to be fixed to the container by a covalent bond.

(3) Quantitative determination of unreacted peptide using Ellman's reagent

Then, 10 μL each of a 2-nitro-5-mercaptobenzoic acid (TNB) solution was dispensed. The TNB solution turns yellow upon reaction with a free thiol group. The unreacted RGD peptide (containing cysteine) turned yellow with time and the absorbance was measured for quantitative determination, and the proportion of remaining peptide to the peptide before reaction was calculated. The results are shown in Fig. 1.

(4) Results

It is found from Fig. 1 that in all Ex. 1 to 3, the proportion of the remaining peptide decreased with time. Further, it was evident that in Ex. 2 and 3, the reaction efficiency of the peptide and the maleimide group was more excellent.

[Test Example 4] Test on confirmation of cell adhesion of plate

(1) Preparation of plate coated with polymer

Five types of the polymer F were prepared adjusting the molar ratio of the compound D obtained in production Example 1 and methyl methacrylate. Then, each of the 5 types of the polymer F and the polymer G were mixed in a weight ratio of 10:90 to obtain 5 types of polymer to coat the plate. The amounts of units having a maleimide group contained in the obtained polymers were 0, 0.02, 0.19, 0.95 and 1.85 μmol, respectively.

Then, the respective polymers were dissolved in AK-225 (manufactured by Asahi Glass Company, Limited) so that their concentration would be 0.1 mass% to prepare coating solutions. Each of the prepared coating solutions was dispensed in 4 wells in a longitudinal direction of a 24-well microplate (manufactured by ATG) in an amount of 2.2 mL per well and left to stand for one day to volatilize the solvent thereby to form a coating layer on the well surface. As a control, leftmost 4 wells in a longitudinal direction were untreated.

(2) Maleimide group inactivation treatment

In order to confirm that the maleimide group contained in the polymer specifically fixes protein, as a negative control, a maleimide group inactivation treatment was conducted on the lowermost wells (6 wells) of the 24-well microplate by the following method.

First, a powdery cysteine was dissolved in PBS at a concentration of 10 mg/mL to prepare a cysteine solution. In the cysteine solution, dithiothreitol (DTT) (manufactured by Wako Pure Chemical Industries, Ltd.) was dissolved so that the final concentration would be 100 mM. The DTT-containing cysteine solution was dispensed in the lowermost wells (6 wells) of the 24-well microplate in an amount of 300 μL per well and reacted at room temperature for 3 hours, followed by washing with PBS three times.

(3) Fixation of peptide

Then, RGD peptide (containing cysteine) having cell adhesion activity was reacted with maleimide in the wells at a low concentration (0.02 μmol) and at a high concentration (0.2 μmol) to conduct fixation to the container by a covalent bond.

First, 5 mg of powdery RGD peptide (containing cysteine) was dissolved in 724 μL of PBS to prepare a 10 mM peptide solution. The solution was diluted 20-fold with PBS to prepare a high concentration RGD peptide (containing cysteine) solution and diluted 200-fold with PBS to prepare a low concentration RGD peptide (containing cysteine) solution. The low concentration RGD peptide (containing cysteine) solution was dispensed in the second top wells (6

wells) and the high concentration RGD peptide (containing cysteine) solution was dispensed in the third and fourth top wells (12 wells) in an amount of 400 μL per well and reacted at 4°C overnight. On that occasion, the amount of the peptide contained in one well into which the low concentration RGD peptide (containing cysteine) solution was dispensed was 0.02 μmol, and the amount of the peptide contained in one well into which the high concentration RGD peptide (containing cysteine) solution was dispensed was 0.2 μmol. Then, the supernatant was removed, followed by washing with PBS three times.

(4) Unreacted maleimide group inactivation treatment

In order to prevent bonding of the maleimide group to which the RGD peptide (containing cysteine) was not fixed, to cell secretory factors or cells themselves, before inoculation of cells, a treatment to inactivate unreacted maleimide groups remaining in the polymer was carried out.

First, 400 μL of the DTT-containing cysteine solution prepared in (1) was dispensed to each of all the 24 wells and reacted at room temperature for 3 hours, followed by washing with PBS three times.

(5) Inoculation of cells

In order to confirm cell adhesion, cell adhesion assay using TIG-3 cells was carried out. TIG-3 cells are fibroblast cells derived from a 10-week old Japanese male fetal lung with trypsin method. The TIG-3 cells were ones subcultured at 37°C in a CO2 incubator with 5% carbon dioxide ventilation using an MEM medium (manufactured by Thermo Fisher Scientific Inc.) containing 10% fetal bovine serum (manufactured by Thermo Fisher Scientific Inc.) (hereinafter referred to as maintenance medium).

First, TIG-3 cells cultivated in a 100 mm dish were washed with 5 mL PBS. Then, 1 mL of Trypsin/EDTA (manufactured by Thermo Fisher Scientific Inc.) as a cell dissociation reagent was added, followed by reaction at 37°C for 3 minutes. Then, 9 mL of the maintenance medium was added to dilute the dissociation reagent thereby to terminate the reaction. The mixture was put in a 15 mL centrifugal tube and subjected to centrifugal separation (160 × g, 5 min). Then, the supernatant was removed, and the precipitate was suspended in 5 mL of an MEM medium containing no serum, and centrifugal separation was carried out again (160 × g, 5 min). In order to remove the serum component, washing was carried out twice with the MEM medium containing no serum. Then, the precipitate was suspended in an MEM medium, and the number of cells was counted and adjusted to be $5\times10^5$ cells/mL. Then, the cell suspension was inoculated in wells of the 24-well microplate in an amount of 300 μL per well and left at rest in a $CO_2$ incubator at 37°C for one hour. Then, the culture supernatant was removed, and an MEM medium containing Cell Counting Kit-8 (manufactured by DOJINDO LABORATORIES) in an amount of one tenth of the entire amount was dispensed in the wells in an amount of 300 μL per well. One hour later, the culture supernatant was recovered, and using 100 μL thereof, the absorbance at 450 nm was measured. Employing the obtained absorbance, the number of cells attached was quantitatively determined. The results are shown in Figs. 2 and 3.

(6) Results

From Fig. 2, a tendency such that many cells were attached to the untreated wells in the plate (see the "untreated" wells in a longitudinal direction in Fig. 2) regardless of presence or absence of the RGD peptide (containing cysteine) was observed. Whereas in wells coated with a polymer containing no maleimide group (see the wells with an amount of maleimide group contained in polymer of "0" in a longitudinal direction in Fig. 2), substantially no adhesion of cells was observed, and thus a cell adhesion suppression effect which is a basic characteristic of the polymer could be confirmed.

Further, from Fig. 3, a tendency such that the number of cells attached to the well having the RGD peptide (containing cysteine) fixed, increased in correlation with the amount of the maleimide group contained in the polymer was confirmed (see RGD (low concentration) and RGD (high concentration) in Fig. 3). Such a tendency was not observed with respect to wells in which the maleimide group was inactivated with a cysteine solution (see RGD (maleimide inactivated) in Fig. 3), and it was indicated that the cells were attached to the peptide and the peptide was fixed via the maleimide group contained in the polymer.

Further, from Fig. 3, when the amount of the maleimide group was 0.02 μmol, no dependence on the concentration of the peptide was observed. This is estimated to be because binding of the peptide to the maleimide group is saturated.

Further, when the amount of the maleimide group was 0.19 μm, cell adhesion activity dependent on the concentration of the peptide was observed. This is estimated to be because the maleimide group is present in a sufficient amount.

Further, when the amount of the maleimide group was 0.95 or 1.85 μmol, the absorbance increased with respect

to the untreated wells and the wells in which the maleimide group was inactivated. This is estimated to be because the amount of the maleimide group is large and the proportion of the group having biocompatibility (a polyethylene glycol group in this Test Example) relatively decreases, and accordingly protein derived from cells is non-specifically adsorbed.

Accordingly, it is suggested that optimally the RGD peptide (containing cysteine) in an amount of 0.2 μmol and the maleimide group in an amount of 0.19 μmol are bonded, and that the amount of the aimed protein and the amount of the fixing group are preferably at the same level.

Fig. 4 is images illustrating comparison of the forms of the TIG-3 cells between in the well to which the high concentration RGD peptide (containing cysteine) was fixed and in the untreated well. From Fig. 4, in the well to which the high concentration RGD peptide (containing cysteine) was fixed, the TIG-3 cells had a round cell form and were clearly defined. Whereas in the untreated well, the TIG-3 cells had a flat form and significantly extended. This difference is estimated to be a difference between the cells being attached at a "point" and on a "plane".

From the above results, it was strongly suggested that the polymers in Examples of the present invention have a function in accordance with the concept of "selective partial adhesion".

## INDUSTRIAL APPLICABILITY

The resin composition of the present invention is suitable as a coating material for a substrate to selectively trap a target substance, and is particularly suitable as a coating material for a substrate for cell culture to selectively trap specific cells and incubate them in a state such that inclusion of other proteins, etc. is prevented as far as possible.

The entire disclosure of Japanese Patent Application No. 2015-251518 filed on December 24, 2015 including specification, claims, drawings and summary is incorporated herein by reference in its entirety.

## Claims

**1.** A resin composition comprising a polymer having units (a) having at least one functional group selected from the group consisting of a maleimide group, a succinimide group, a thiol group and a hydrazino group, and units (b) having at least one group selected from the group consisting of a group represented by the following formula (1), a group represented by the following formula (2) and a group represented by the following formula (3) or comprising a polymer (A) having the units (a) and a polymer (B) having the units (b):

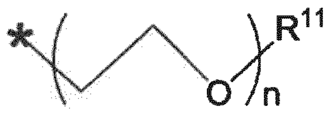

(1)

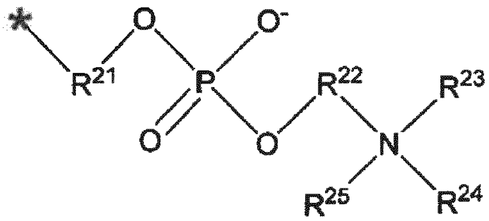

(2)

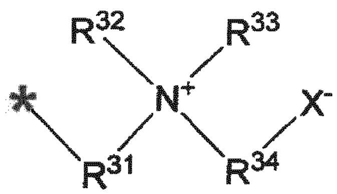

(3)

wherein * is a direct binding site to the polymer main chain or an indirect binding site via a linking group; n is an integer of from 1 to 10, $R^{11}$ is a hydrogen atom, a methyl group or an ethyl group; $R^{21}$ and $R^{22}$ are each independently a $C_{1-5}$ alkylene group, and $R^{23}$ to $R^{25}$ are each independently a $C_{1-5}$ alkyl group; $R^{31}$ is a $C_{1-20}$ alkylene group, $R^{34}$ is a $C_{1-5}$ alkylene group, $R^{32}$ and $R^{33}$ are each independently a $C_{1-5}$ alkyl group, and $X^-$ is a group represented by the following formula (4) or a group represented by the following formula (5):

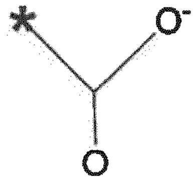 (4) 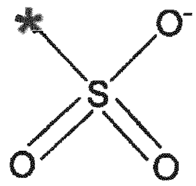 (5)

wherein * is a binding site to $R^{34}$.

**2.** The resin composition according to Claim 1, which further contains a polymer (C) having units (c) having a group represented by the following formula (6), or
wherein the polymer having the units (a) and the units (b) has units (c) having a group represented by the following formula (6), or at least one of the polymer (A) and the polymer (B) has units (c) having a group represented by the following formula (6):

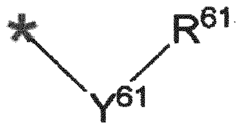 (6)

wherein * is a direct binding site to the polymer main chain, $Y^{61}$ is a single bond or a bivalent organic group, and $R^{61}$ is a $C_{1-20}$ alkyl group.

**3.** The resin composition according to Claim 1 or 2, wherein in the polymer having the units (a) and the units (b) or in the polymer (A), the proportion of the units (a) is from 0.001 to 5 mol% to the total number of units constituting the composition.

**4.** The resin composition according to any one of Claims 1 to 3, wherein in the polymer having the units (a) and the units (b) or in the polymer (B), the proportion of the units (b) is from 5 to 60 mass% to the total number of units constituting the composition.

**5.** The resin composition according to any one of Claims 2 to 4, wherein the units (c) having a group represented by the formula (6) are contained in a proportion of from 30 to 90 mass% to the total number of units constituting the composition.

**6.** The resin composition according to any one of Claims 2 to 5, which further contains units (d) having a crosslinkable group selected from a functional group which forms a silanol group by hydrolysis, an epoxy group, a (meth)acrylic group and a glycidyl group.

**7.** The resin composition according to Claim 6, which contains from 0.002 to 3 mass% of the units (a), from 10 to 45 mass% of the units (b), from 50 to 80 mass% of the units (c) and from 0 to 2.5 mass% of the units (d).

**8.** A substrate which has at least a part of its surface coated with the resin composition as defined in any one of Claims 1 to 7.

**9.** The substrate according to Claim 8, which is for cell culture.

**10.** A cell culture process, which comprises a step of bonding a ligand having a moiety which specifically binds to the surface of target cells, to the at least one functional group selected from the group consisting of a maleimide group, a succinimide group, a thiol group and a hydrazino group on the surface of the substrate as defined in Claim 8,
a step of bringing the target cells into contact with the substrate having the ligand bonded thereto, to bond the target cells to the ligand, and
a step of incubating the target cells bonded to the ligand.

Fig. 1

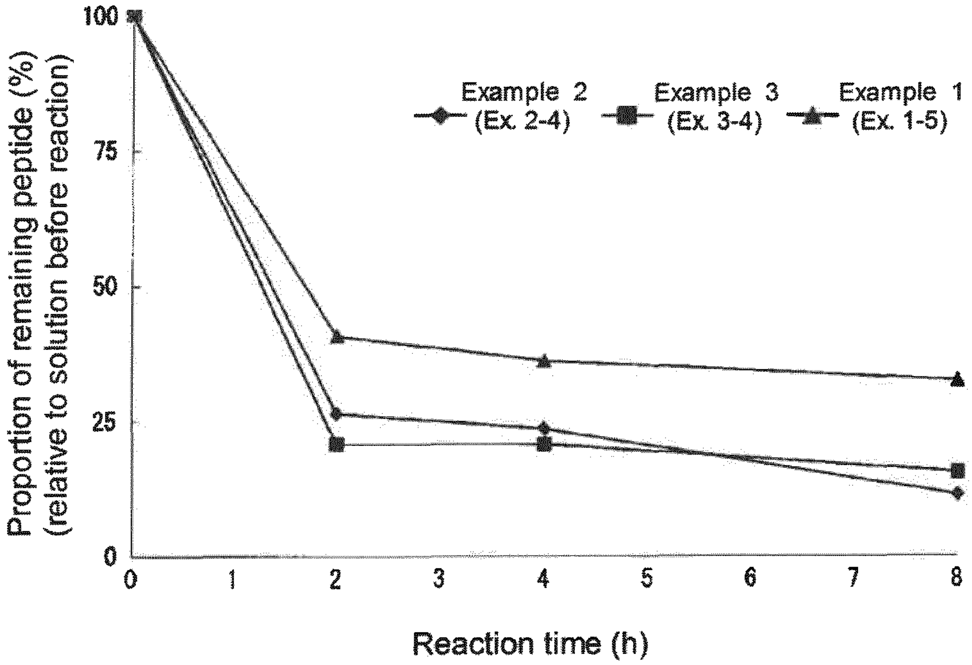
100
75
50
25
0
Proportion of remaining peptide (%)
(relative to solution before reaction)
Example 2 (Ex. 2-4)
Example 3 (Ex. 3-4)
Example 1 (Ex. 1-5)
0
1
2
3
4
5
6
7
8
Reaction time (h)

Fig. 2

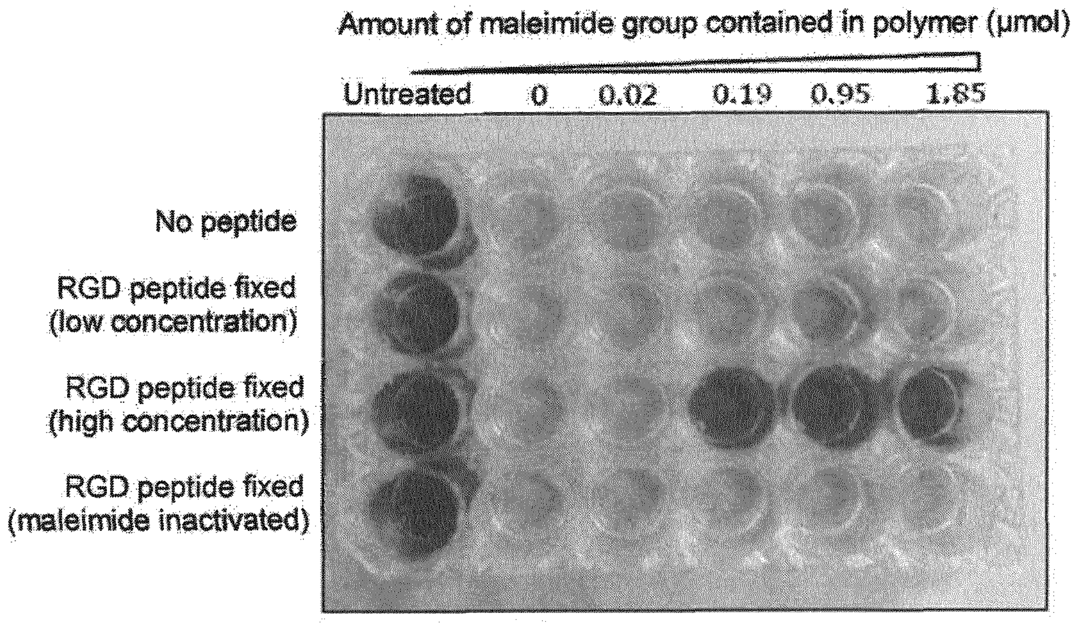
Amount of maleimide group contained in polymer (μmol)
Untreated
0
0.02
0.19
0.95
1.85
No peptide
RGD peptide fixed
(low concentration)
RGD peptide fixed
(high concentration)
RGD peptide fixed
(maleimide inactivated)

Fig. 3

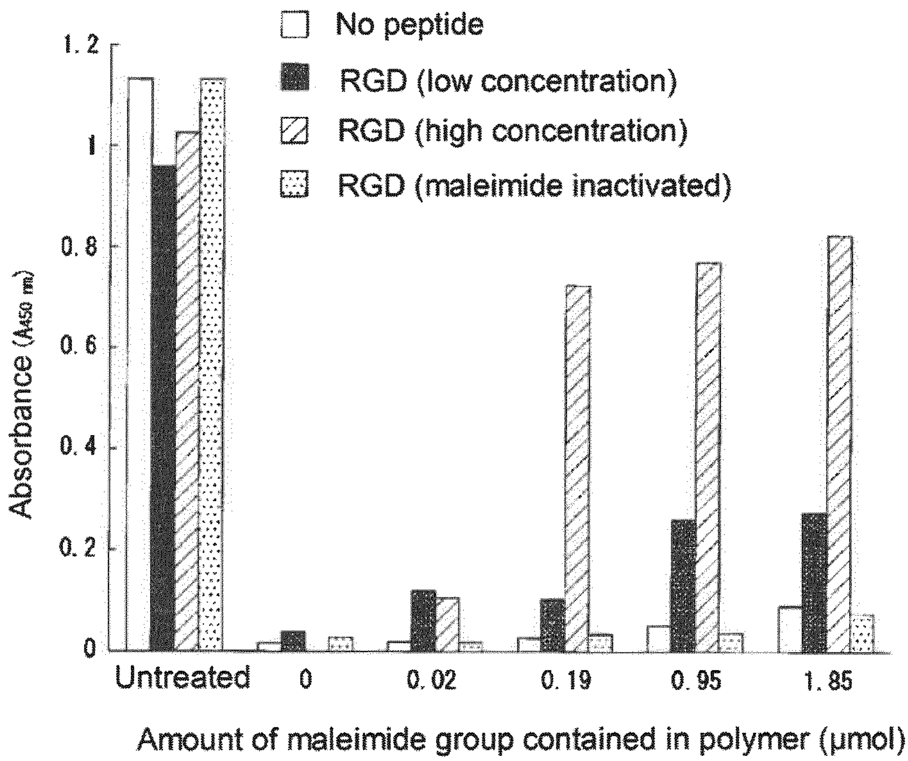
No peptide
RGD (low concentration)
RGD (high concentration)
RGD (maleimide inactivated)
Absorbance (A450 nm)
1.2
1
0.8
0.6
0.4
0.2
0
Untreated
0
0.02
0.19
0.95
1.85
Amount of maleimide group contained in polymer (µmol)

Fig. 4

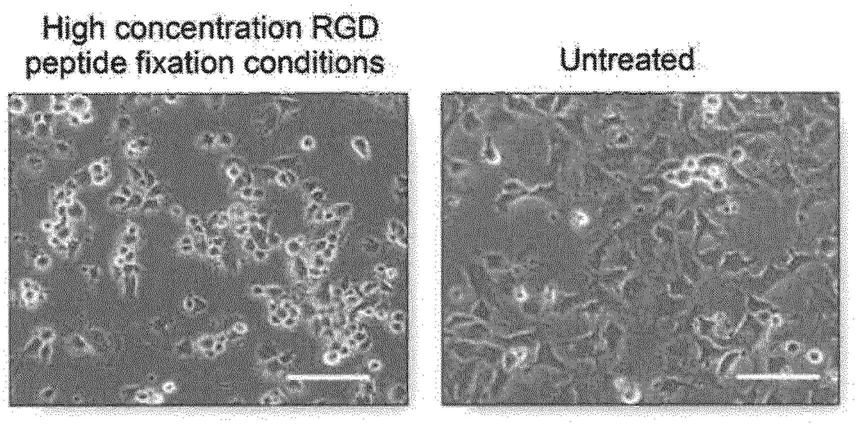
High concentration RGD peptide fixation conditions
Untreated

**INTERNATIONAL SEARCH REPORT**

International application No. PCT/JP2016/088209

A. CLASSIFICATION OF SUBJECT MATTER
*C08L101/02*(2006.01)i, *C12M3/00*(2006.01)i, *C12N5/07*(2010.01)i

According to International Patent Classification (IPC) or to both national classification and IPC

B. FIELDS SEARCHED

Minimum documentation searched (classification system followed by classification symbols)
C08L101/02, C12M3/00, C12N5/07

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2006-176720 A (Sumitomo Bakelite Co., Ltd., Kazuhiko ISHIHARA), 06 July 2006 (06.07.2006), claims; paragraphs [0008] to [0012]; examples (Family: none) | 1-8<br>9-10 |
| X<br>A | JP 2-272559 A (Fuji Photo Film Co., Ltd.), 07 November 1990 (07.11.1990), example 17 (Family: none) | 1-2,8<br>3-7,9-10 |
| X<br>A | JP 2001-146556 A (Nano Carrier Co., Ltd.), 29 May 2001 (29.05.2001), claims & WO 2001/037879 A1 & EP 1230934 A1 claims & CA 2391964 A1 | 1<br>2-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 March 2017 (09.03.17) | 21 March 2017 (21.03.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2016/088209

C (Continuation). DOCUMENTS CONSIDERED TO BE RELEVANT

| Category* | Citation of document, with indication, where appropriate, of the relevant passages | Relevant to claim No. |
|---|---|---|
| X<br>A | MATHEW P. Robin, RACHEL K. O'Reilly, Fluorescent and chemico-fluorescent responsive polymers from dithiomaleimide and dibromomaleimide functional monomers, Chemical Science, 2014.05.16, Issue7, p.2717-2723 | 1<br>2-10 |
| A | JP 7-504459 A (Biocompatibles Ltd.), 18 May 1995 (18.05.1995), & US 5712326 A & WO 1994/014897 A1 | 1-10 |
| A | JP 3-59666 A (Fuji Photo Film Co., Ltd.), 14 March 1991 (14.03.1991), (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2011510655 A
- JP 2015251518 A

### Non-patent literature cited in the description

- **XU, CHUNHUI. et al.** Feeder-free growth of undifferentiated human embryonic stem cells. *Nat. Biotech.,* 2001, vol. 19 (10), 971-979